# EUROPÄISCHE PATENTSCHRIFT

(11) **EP 0 934 053 B1**
(45) Veröffentlichungstag und Bekanntmachung des Hinweises auf die Patenterteilung: **03.03.2004**
(21) Anmeldenummer: 97912150.6
(22) Anmeldetag: 09.10.1997
(51) Int. Cl.: A61K 7/00

(54) **KOSMETISCHE ODER DERMATOLOGISCHE MIKROEMULSION**
COSMETIC OR DERMATOLOGICAL MICROEMULSIONS
MICROEMULSIONS COSMETIQUES OU DERMATOLOGIQUES

(30) Priorität: 10.10.1996 DE 19641672
(43) Veröffentlichungstag der Anmeldung: 11.08.1999
(73) Patentinhaber: Beiersdorf Aktiengesellschaft, 20245 Hamburg (DE)
(72) Erfinder: DIEC, Khiet, Hien, D-22523 Hamburg (DE); MEIER, Wolfgang, CH-4053 Basel (CH); SCHREIBER, Jörg, D-22087 Hamburg (DE)
(86) Internationale Anmeldenummer: PCT/EP1997/005553
(87) Internationale Veröffentlichungsnummer: WO 1998/015255

(56) Entgegenhaltungen:
- EP-A- 0 278 660
- EP-A- 0 316 726
- EP-A- 0 572 080
- EP-A- 0 728 460
- WO-A-96/28132
- DE-A- 4 417 476
- US-A- 4 052 331

## Beschreibung

Die vorliegende Erfindung betrifft Mikroemulsionsgele vom Typ Öl in Wasser, Verfahren zu ihrer Herstellung sowie ihre Verwendung für kosmetische Zwecke, insbesondere zur topischen Anwendung.
Als besondere Ausführungsform betrifft die vorliegende Erfindung die Verwendung erfindungsgemäßer Mikroemulsionsgele für medizinische Zwecke, insbesondere als Arzneistoffträger für lipophile Wirkstoffe und ihren pharmazeutischen Einsatz als topische Dermatika, aber auch zur parenteralen Verabreicherung pharmazeutischer Wirkstoffe und zur parenteralen Ernährung.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche, und sich gerade in neuerer Zeit immer weiter verbreitende kosmetische und dermatologische Zubereitungsformen sind Gele.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind und Öl/Wasser-Gele, welche letztlich auf O/W- oder W/O-Emulsionen basieren, in welchen zusäzlich aber auch Merkmale einer Gelstruktur verwirklicht sind. Meistens sind Gele durchsichtig. In der kosmetischen bzw. pharmazeutischen Galenik zeichnen sich Gele in aller Regel durch halbfeste, oft fließfähige Konsistenz aus.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweißgefärbt und undurchsichtig.

Mizellaren und molekularen Lösungen mit Partikeldurchmessern kleiner als ca. 10⁻² µm, ist vorbehalten, klar und transparent zu erscheinen.

Der Tröpfchendurchmesser von transparenten bzw. transluzenten Mikroemulsionen dagegen liegt im Bereich von etwa 10⁻² µm bis etwa 10⁻¹ µm. Solche Mikroemulsionen sind meist niedrigviskos. Die Viskosität vieler Mikroemulsionen vom O/W-Typ ist vergleichbar mit der des Wassers.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%. bezogen auf die Gesamtzusammensetzung. Solubilisiert man in diese Tensidgele, fachsprachlich auch "surfactant gels" genannt, Ölkomponenten, werden Mikroemulsionsgele erhalten, weiche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten. Auch hier ist der hohe Gehalt an Emulgatoren von Nachteil.

Vorteil von Mikroemulsionsgelen ist, daß in der dispersen Phase Wirkstoffe feindispers vorliegen können. Ein weiterer Vorteil ist, daß sie aufgrund ihrer niedrigen Viskosität versprühbar sein können. Werden Mikroemulsionen als Kosmetika verwendet, zeichnen sich entsprechende Produkte durch hohe kosmetische Eleganz aus.

Es ist an sich bekannt, die Tröpfchen einer niedrigviskosen, insbesondere dünnflüssigen Mikroemulsion mit vemetzenden Substanzen miteinander zu verknüpfen, um auf diese Weise das dreidimensionale Netzwerk eines Geles zu erhalten.

In Nachr. Chem. Techn. Lab. 43 (1995) Nr. 1, S. 9 ff. werden zur Vernetzung von Mikroemulsionströpfchen kettenförmige, hydrophile Moleküle beschrieben, welche an den beiden Kettenenden je einen hydrophoben Rest aufweisen. Jene hydrophoben Reste tauchen in Mikroemulsionströpfchen ein, wobei die hydrophilen Kettenbereiche in der kontinuierlichen Wasserphase vorliegen. Im strengen Sinne ist es wohl nicht nötig, daß die hydrophoben Reste "eintauchen". Es kann im Einzelfalle auch durchaus genügen, wenn durch hydrophobe Wechselwirkung die hydrophoben Reste mit der Oberfläche der Mikroemulsionströpfchen in Kontakt treten und mehr oder weniger stark an dieser haften bleiben.

Als Vernetzer werden a.a.O. Polyoxyethylenglycole mit Oleylgruppen als hydrophobe Endgruppen angegeben.

Nachteilig an Mikroemulsionen, und somit auch an den Mikroemulsionsgelen des Standes der Technik ist, daß stets ein hoher Gehalt an einem oder mehreren Emulgatoren eingesetzt werden muß, da die geringe Tröpfchengröße eine hohe Grenzfläche zwischen den Phasen bedingt, welche in der Regel durch Emulgatoren stabilisiert werden muß.

WO 96/28132 offenbart Mikroemulsionsgele OW die durch Phasenumkehr aus WO Emulsion durch Erwärmen und Abkühlen erhältlich sind und polyethoxylierte - bzw. polypropoxylierte O/W Emulgatoren enthalten.

An sich ist die Verwendung der üblichen kosmetischen Emulgatoren zwar unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, Sonnenschutzprodukte zu entwickeln.

So betrifft die vorliegende Erfindung als besondere Ausführungsformen kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird im allgemeinen der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasem des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Mikroemulsionsgele eignen sich auch für andere kosmetische dermatologische Anwendungen, beispielsweise Desodorantien, so daß die vorliegende Erfindung in einer besonderen Ausführungsform Mikroemulsionsgele als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird, Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Auch die Verwendung von Mikroemulsionen als Grundlage für desodorierende oder antitranspirant wirkende Zubereitungen sind bekannt. Deren relativ hoher Gehalt an Emulgatoren, mit den geschilderten Nachteilen, war bisher ein Übelstand, dem es abzuhelfen galt.

Eine weitere Aufgabe der vorliegenden Erfindung war es also, Zubereitungen zu entwikkein, welche als Grundlage für kosmetische Desodorantien bzw. Antitranspirantien geeignet sind, und die Nachteile des Standes der Technik nicht aufweisen.

Weiterhin war es eine Aufgabe der Erfindung, kosmetische Grundlagen für kosmetische Desodorantien zu entwickeln, die sich durch gute Hautverträglichkeit auszeichnen.

Ferner war eine Aufgabe der vorliegenden Erfindung, Produkte auf der Basis von Mikroemulsionsgelen mit einer möglichst breiten Anwendungsvielfalt zur Verfügung zu stellen. Beispielsweise sollten Grundlagen für Zubereitungsformen wie Reinigungsemutsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

In einer besonderen Ausführungsform betrifft die Erfindung daher Reinigungsemulsionen, insbesondere Gesichtsreinigungsemulsionen, bevorzugt Make-up-Entferner, beispielsweise Augenmake-up-Entferner.

Solche Zubereitungen sind an sich bekannt. Üblicherweise handelt es sich dabei um Abmischungen kosmetischer öle oder wäßrige. Zubereitungen oberflächenaktiver Substanzen, deren Funktion darin besteht, die Verunreinigung oder den Make-up-Körper zu solubilisieren und von der Haut zu entfernen.

Wasserfestes Augen-Make-up, beispielsweise Mascara, ist mit Make-up-Entfernern auf wäßriger Basis nur mit speziellen Tensiden zufriedenstellend zu entfernen. Diese Tenside besitzen aber oft eine nur begrenzte physiologische Verträglichkeit. Bei einem Kontakt solcher Stoffe mit der Schleimhaut, insbesondere der Augenschleimhaut, führen diese Stoffe zu Reizungen, die sich beispielsweise in einer Rötung der Augen äußem. Reaktionen dieser Art sind typisch für tensidhaltige Produkte.

Eine Aufgabe der vorliegenden Erfindung war mithin, solchen Problemen Abhilfe zu schaffen.

Die vorliegende Erfindung betrifft in einer weiteren Ausführungsform haarkosmetische Zubereitungen. Insbesondere betrifft die vorliegende Erfindung haarkosmetische Zubereitungen zur Pflege des Haars und der Kopfhaut. In einer bevorzugten Ausführungsform betrifft die vorliegende Erfindung Zubereitungen, die dazu dienen, das einzelne Haar zu kräftigen und/oder der Haartracht insgesamt Halt und Fülle zu verleihen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt, ferner dem die Medulla umschließenden Cortex und der die Gesamtheit aus Medulla und Cortex umhüllenden Cuticula.

Insbesondere die Cuticula, aber auch der keratinöse Bereich zwischen Cuticula und Cortex als Außenhülle des Haares sind besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Hamsäure, Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stand der Technik ließ es aber an Wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Aufgabe war daher, auch diesen den Nachteilen des Standes der Technik Abhilfe zu schaffen.

Eine besondere Aufgabe der vorliegenden Erfindung war es, gelartige Zubereitungen auf Basis feindisperser Systeme vom Typ Öl-in-Wasser mit einem möglichst niedrigen Emulgatorgehalt zur Verfügung zu stellen, welche nicht die Nachteile des Standes der Technik aufweisen und welche für verschiedenste kosmetische und/oder dermatologische Anwendungen, beispielsweise die vorab beschreibenen Verwendungen finden können. Eine weitere Aufgabe der Erfindung war, das begrenzte Angebot an gelartigen Zubereitungen auf Basis feindisperser Systeme vom Typ Öl-in-Wasser des Standes der Technik zu bereichem.
In der Offenlegungsschrift WO 96/28132 werden zur Herstellung von Mikroemulsionsgelen ethylenoxidhaltige O/W Emulgatoren eingesetzt, die mit geeigneten W/O-Emulgatoren und einer Ölphase gemischt werden.
Die Herstellung der Gele erfolgt mit Vemetztersubstanzen in der Weise, daß die Mischung zum Beispiel bei höherer Temperatur eine Phaseninversion durchläuft und auf Raumtemperatur abgekühlt wird. Es wird dort ferner beschrieben, daß die Vernetzung der Öltöpfchen bzw. die Herstellung der Mikroemulsionsgele auch ohne Phaseninversion gelingt.

Auf solche Weise hergestellte Mikroemulsionen des Standes der Technik haben allerdings den Nachteil, daß das Verfahren auf ethylenoxidhaltigen Emulgatoren beruht.
Aus ökologischen Gesichtspunkten und der bekannten Tatsache, daß EO-haltige Emulgatoren aufgrund von Verunreinigungen beim Herstellprozeß hautunverträglich sein können, sollten diese Nachteile des Standes der Technik beseitigt werden.

Es ist an sich bekannt, niedrigviskose Mikroemulsionen ohne diese ethylenoxidhaltigen Emulgatoren herzustellen. In DE4417476 A1 werden Mikroemulsionen mit Alkylpolyglycosiden (Plantaren 1200) beschrieben, die als Coemulgatoren Fettsäure-Polyol-partialester enthalten. Ferner werden dort Alkylpolyglycosid/ethylenoxidhaltige Emulgator-Mischungen beschrieben, die es gestatten Kohlenwaserstoffe zu solubilisieren. Die Transparenz der Mikroemulsionen ist bekanntlich dann am größten, wenn es gelingt, die Tröpfchengröße unter 100 nm zu halten (siehe Tabelle III, Beispiel 16).
Nachteilig ist hier, daß für die Herstellung von APG-haltigen Mikroemulsionen ausschließlich Kohlenwasserstoffe geeignet sind.
Eine Erklärung für diese Tatsache findet sich in einer Publikation in Colloid & Polymer Science 273:1995, S 565ff. Polarere Öle als Kohlenwasserstoffe führen dazu, daß die Alkylpolyglycoside von der Öl/Wasser Grenzfläche abwandern. Da nur die an der Grenzfläche vorhandenen Emulgatoren eine Reduktion der Öl-Wasser-Grenzflächenspannung auf Werte um O mN/m⁻¹ bewirken können, werden daher Kohlenwasserstoffe als Ölkomponenten bevorzugt zur Herstellung APG-haltiger Mikroemulsionen eingesetzt.

Ferner werden in DE 4411557 A1 u.a. niedrigviskose Mikroemulsionen beschrieben, die aus einer Mischung von ethylenoxidhaltigen nichtionischen und anionischen Tensiden bestehen (Ceteth-5/Oleth-5 ; Laurylether-2-sulfat) bestehen.
Niedrigviskose Mikroemulsionen für orale Anwendungen auf Basis von Lecithin/ Ethanol/ Propylenglycol werden in WO 92/02207 beschrieben. Die Verdickung zum Mikroemulsionsgel wird mit Gelatine als wasserlöslichem Polymer induziert. Nachteil für kosmetische Anwendungen ist das Fehlen einer kosmetischen Ölphase. Ferner wird das erfindungsgemäße Prinzip der Vernetzung von Öltröpfchen durch hydrophob modifizierte wasserlösliche Polymere nicht angewendet.
Lecithingele werden in der Literatur (J. Phys. Chem. 92, 1988, 829; Colloid Polymer Science 268, 1990, 356) beschrieben. Diese Gele erhält man durch Zugabe kleiner Mengen an Wasser zu einer Mischung aus organischem Solvens und Lecithin. Aus inversen Micellen entstehen bei Wasserzugabe zylinderartige, wassersolubilisierende Strukturen, die miteinander verschlaufen und so die hohe Viskosität dieser Mischungen erklären. (Colloid Poly. Sci. 268, 1990, 356). Der Einsatz von Ethanol als amphiphiles Cosolvens zur Herstellung von lecithinhaltigen Mikroemulsionen sowie die Gelierung mit Polysacchariden wie Gelatine oder Agar wird auch in WO 95/31969 beschrieben. Das erfindungsgemäße Prinzip der Vernetzung von Mikroemulsionströpfchen durch hydrophob modifizierte wasserlösliche Polymere wird nicht erläutert. Ferner wird nicht beschrieben, daß durch Zusatz eines Coemulgators vom Typ Öl-in-Wasser zu einer Lecithin/Öl-Mischung bei Wasserzugabe unter Ausnutzung eines intermediär gebildeten viskosen Gelzustands bei weiterer Wasserzugabe sich erfindungsgemäße Öl-in-Wasser-Mikroemulsionen oder bei Anwesenheit erfindungsgemäßer Vemetzer erfindungsgemäße Öl-in-Wasser-Mikroemulsionsgele erhalten lassen. Ferner war dem Stande der Technik bislang nicht bekannt, wie wenigstens ein gewisser Anteil der in Körperflüssigkeiten vorhandenen freien Zellen oder gegebenenfalls auch mehr oder weniger agglomeriert vorkommenden Zellen mit einander verknüpft werden können, wodurch der betreffenden Körperflüssigkeit eine Viskositätserhöhung zuteil werden könnte. Bei Wunden des menschlichen oder tierischen Körpers erfolgt zunächst der Austritt einer mehr oder weniger großen Menge Blutes, dessen Gerinnung eine wichtige Rolle bei der Wundheilung spielt. Bis zur Gerinnung liegt das Blut allerdings recht niedrigviskos vor, so daß abfließendes Blut lediglich den Körper durch Volumenmangel schwächt, zur Wundheilung indes keinen Beitrag liefert. Wünschenswert wäre, dem Blute lokal, also extrakorporal zu einer Viskositätserhöhung zu verhelfen, um dem Volumenmangel, welcher im übrigen, zumal bei stark blutenden Wunden zum lebensbedrohenden Volumenmangelschock führen kann, vorzubeugen.

Ferner war nicht bekannt, daß zum Beispiel Emulgatoren auf Basis von Acyllactylaten und Acylglutamaten oder auch nichtionische ethylenoxidfreie Emulgatoren geeignet sind, in Gegenwart geeigneter ethylenoxidfreier Coemulgatoren und/oder Lecithin transparente Mikroemulsionsgele zu geben. Acyllactylate als Emulgatoren für Makroemulsionen sind hingegen bekannt (Food Prod. Developm. 6, 1972, 80-84; WO 88/06880, DE 4412081). Ferner ist auch in den Schriften WO 95/05799 und EP 573253 beschrieben, daß zum Beispiel Acyllactylate antibakteriell wirksam sein können oder anderweitig vorteilhaft (WO 95/05153; US 3472940; EP 586234) sind.

Zusammenfassend war es die Aufgabe der vorliegenden Erfindung, ethylenoxidfreie und propylenoxidfreie Emulgatoren in niedriger Konzentration zur Herstellung von transparenten/ translucenten Mikroemulsionen bzw. -gelen mit einer breiteren Variationsmöglichkeit kosmetischer Ölkomponenten zugänglich zu machen. Ferner war es die Aufgabe der vorliegenden Erfindung Emulgatoren bzw. Polymere einzusetzen, die neben ihrer Eigenschaft, die Grenzflächenspannung zu reduzieren bzw. eine Verdickung der Mikroemulsion zu einem Gel zu bewirken, zusätzlich eine physiologische Wirkung zu erzielen.

Erstaunlicherweise werden alle der Erfindung zugrundliegenden Aufgaben gelöst durch Mikroemulsionsgele,
(a) beruhend auf Mikroemulsionen vom Typ Öl-in-Wasser, welche umfassen
   - eine Ölphase, welche im wesentlichen aus schwerflüchtigen Bestandteilen zusammengesetzt ist, und eine Wasserphase
   - enthaltend:
      einen oder mehrere ethylenoxidfreie und propylenoxidfreie O/W-Emulgatoren und
   - gewünschtenfalls ferner enthaltend einen oder mehrere W/O-Emulgatoren
   - einen Emulgatorgehalt kleiner als 20 Gew.-%, bezogen auf das Gesamtgewicht der Mikroemulsion, aufweisend,
   - erhältlich auf die Weise, daß man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe in ein definiertes Mischungsverhältnis zueinander setzt, so daß eine Mikroemulsion erhalten wird,
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

Erstaunlicherweise werden ferner alle der Erfindung zugrundliegenden Aufgaben gelöst durch Mikroemulsionsgele,
(a) beruhend auf Mikroemulsionen vom Typ Öl-in-Wasser, welche umfassen
   - eine Ölphase, welche im wesentlichen aus schwerflüchtigen Bestandteilen zusammengesetzt ist,
   - enthaltend:
      einen oder mehrere ethylenoxidfreie und propylenoxidfreie O/W-Emulgatoren und Lecithin oder Lecithinderivate und
   - gewünschtenfalls ferner enthaltend einen oder mehrere W/O-Emulgatoren
   - einen Emulgatorgehalt kleiner als 20 Gew.-%, bezogen auf das Gesamtgewicht der Mikroemulsion, aufweisend,
   - erhältlich auf die Weise, daß man ein Gemisch aus den Grundkomponenten, umfassend Ölphase, einen oder mehrere der erfindungsgemäßen O/W-Emulgatoren und Lecithin, gewünschtenfalls einen oder mehrere W/O-Emulgatoren, sowie gewünschtenfalls weitere Hilfs-, Zusatz- und/oder Wirkstoffe langsam mit Wasser versetzt, so daß intermediär ein Gel erhalten wird, daß bei weiterer Wasserzugabe zu einer Mikroemulsion führt,
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

Gegenstand der Erfindung sind auch die niedrigviskosen Mikroemulsionen ohne einen Vernetzeranteil, wie sie vorstehend jeweils unter (a) genannt sind. Sie dienen z.B. als Vorprodukte zur Herstellung der höherviskosen Gele, die mit dem Vernetzer erhalten werden, aber sie können auch wie die jeweils entsprechenden Gele verwendet werden.

Die Herstellungsverfahren von Mikroemulsionen sind an sich bekannt und in der Literatur beschrieben und können auch für die erfindungsgemäßen Mikroemulsionen verwendet werden. Insbesondere werden, wie dem Fachmann bekannt ist, zur Ermittlung des definierten Mischungsverhältnisses die relativen Mengenverhältnisse von Wasser, Ölphase und insbesondere den Emulgatoren (den O/W- und gewünschtenfalls den W/O-Emulgatoren) ermittelt und abgestimmt bis eine klare Mikroemulsion erhalten wird.

Gegenstand der Erfindung ist auch die Verwendung der erfindungsgemäßen Vernetzer zur Herstellung von Mikroemulsionsgelen aus den Mikroemulsionen.

Gegenstand der Erfindung ist auch die Verwendung der erfindungsgemäßen Vernetzer zur Vernetzung oder Verdickung von insbesondere niedrigviskosen Mikroemulsionen.

Gegenstand der Erfindung sind auch die neuen erfindungsgemäßen Vernetzer, insbesondere solche vom Polymertyp, als neue Stoffe.

Die Ausnutzung der intermediär gebildeten Gelphase ist insbesondere vorteilhaft, wenn wärme- oder oxidationsempfindliche oder sonst allgemein empfindliche Wirkstoffe eingesetzt werden sollen (Vitamin-A-Alkohol, Vitamin A-Derivate, Vitamin E und Vitamin E-Derivate, ungesättigte Fettsäuren, Antioxidantien, Lichtschutzfilter usw), da nach der Zugabe dieser Wirkstoffe nur noch die Verdünnung des Gels mit Wasser und gegebenfalls weiterer Zusätze zur Mikroemulsion beziehungsweise bei Anwesenheit der zu jedem Zeitpunkt zusetzbaren Vemetzer die Verdickung der Mikroemulsion zum erfindungsgemäßem Mikroemulsionsgel erfolgt.
Bevorzugte Lecithine und Lecithinderivate bestehen aus Phospholipiden natürlichen, halbsynthetischen und synthetischen Ursprungs. Die Phospholipide können ungesättigt, teilhydriert und hydriert für das erfindungsgemäße Verfahren eingesetzt werden. Beispielsweise vorteilhaft sind auch Phospholipide, die mit weiteren Coemutgatoten oder Ölen versehen sind ("Emulmetik" von Lucas Meyer; "Phosal", "Phospholipon", "Natipide" von Nattermann Phospholipid GmbH, "Lipoid" von Lipod KG).
Hydrierte Phospholipide sind beispielsweise vorteilhaft, wenn auf Antioxidantien verzichtet werden soll. Ferner können auch Sphingolipide wie Sphingosin, Ceramide, Cerebroside, Sphingomyelin erfindungsgemäß verwendet werden. Der Gehalt an Lecithin beziehungsweise an den Derivaten beträgt z.B. 0.001 bis 20 Gew.-%, bezogen auf das Gesamtgewicht der Mikroemulsion.
Für die parenterale Ernährung sind diese auf Lecithin bzw. den Derivaten basierenden Mikroemulsionen sehr gut geeignet, da die Tröpfchengröße im Nanometerbereich liegt und eine Hitzesterilisierung vorgenommen werden kann. Femer sind diese auf Lecithin oder Lecithinderivaten basierenden Mikroemulsionen und Mikroemulsionsgele besonders vorteilhaft für sprühbare Zubereitungen (Sprühgele, nachschäumende Rasiergele, Aerosolsprays), da die enthaltenen Phospholipide sehr gute schaumstabilisierende und hautpflegende Eigenschaften haben.
Es ist dabei gleichermaßen vorteilhaft, wenn die Vernetzersubstanz, im Rahmen der vorliegenden Beschreibung auch als Verdicker oder Verknüpfer oder Verknüpfersubstanz bezeichnet, ein eigenständiges Gelnetzwerk bildet, in welchem die Mikroemulsionströpfchen dann durch hydrophobe Wechselwirkung festgehalten werden (dann liegen sogenannte assoziative Verdicker vor), oder ob der Zusammenhalt des Netz durch die Vernetzung mit den Mikroemulsionströpfchen in den Knotenpunkten des Netzes bewirkt wird.

Die erfindungsgemäß verwendeten Vernetzersubstanzen folgen beispielsweise Strukturschemata wie folgt: 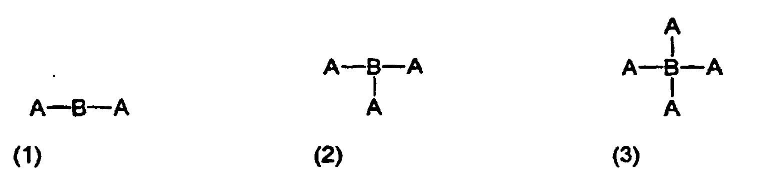 wobei B einen hydrophilen Bereich des jeweiligen Vernetzermoleküls symbolisiert und A jeweils hydrophobe Bereiche, welche auch innerhalb eines Moleküls unterschiedlicher chemischer Natur sein mögen.

Aber auch Strukturschemata wie 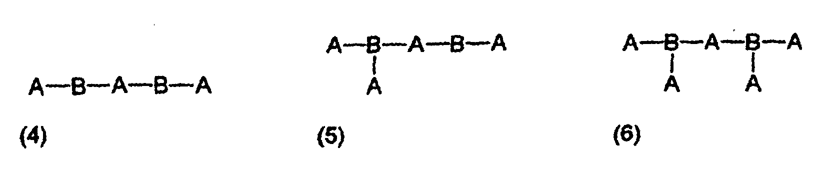 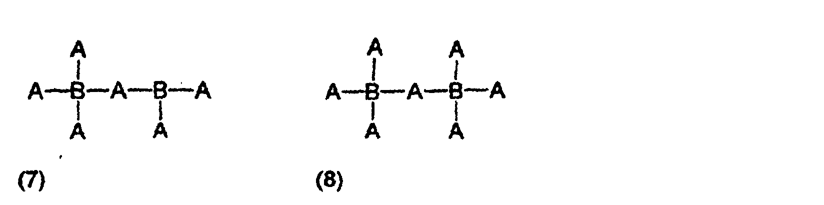 und analog gebildete, noch komplexere Strukturen fallen durchaus in den Rahmen der hiermit vorgelegten Erfindung.

Ebenfalls in den Rahmen der hiermit vorgelegten Erfindung fallen Strukturschemata wie folgt: 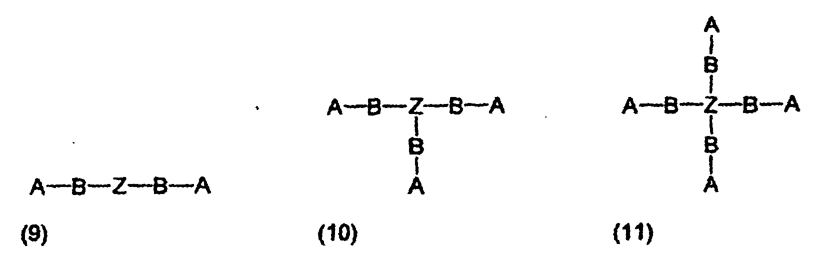 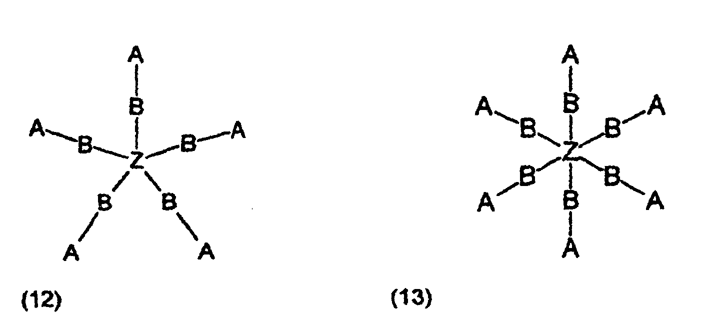 wobei Z dabei eine Zentraleinheit darstellt, welche hydrophil oder hydrophob sein kann und in der Regel aus einem oligo- oder polyfunktionellen Molekülrest besteht. Selbstverständlich fallen auch Verdicker mit höherem Verzweigungsgrad in den Rahmen der vorliegenden Erfindung.

Beispielsweise kann Z in Schema (10) aus einem Glycerylrest bestehen, dessen drei OH-Funktionen in die Bereiche B übergehen, welche ihrerseits beispielsweise Polyoxyethylenketten gleicher oder ungleicher Länge darstellen können, und deren terminale OH-Gruppe mit einer längerkettigen Fettsäure verestert sind. Auch Teilsubstitution an Glycerin ist möglich, wodurch Strukturen entstehen können, welche Schema (9) entsprechen.

Vorteilhaft können die hydrophilen Gruppen B so gewählt werden, daß der Vemetzer insgesamt wasserlöslich oder zumindest in Wasser dispergierbar ist, wobei dann der hydrophobe Anteil der Gruppen A überkompensiert werden sollte.

Für das Strukturschema (1) können beispielsweise folgende spezielleren Strukturschemata befolgt werden: 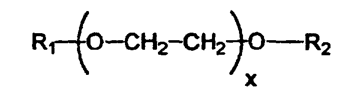 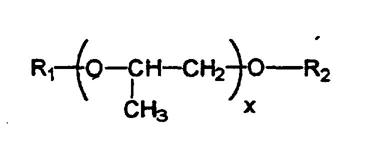 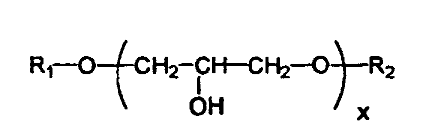 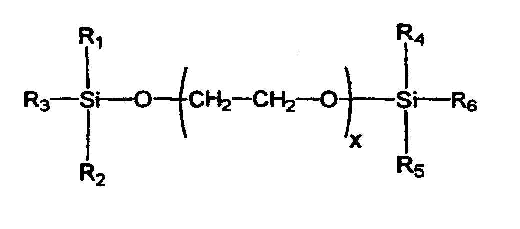 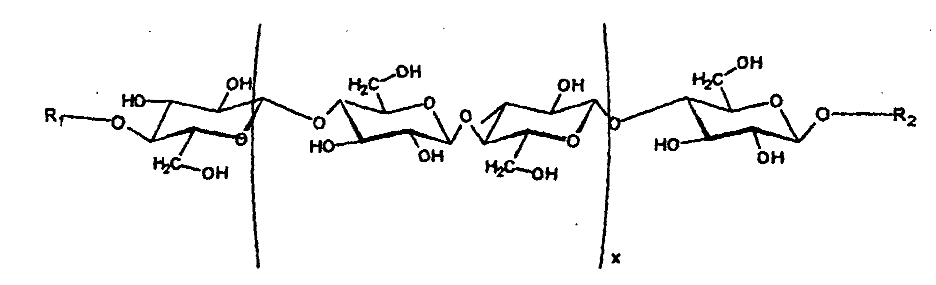 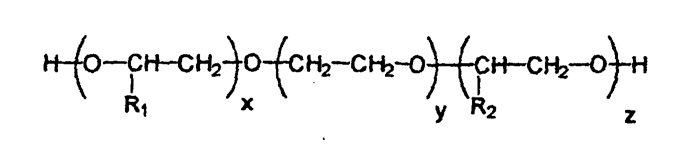 wobei R₁, R₂, R₃, R₄, R₅ und R₆ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyloder Alkanoylreste, mit Alkyl- oder Arylsubstituenten substituierte oder unsubstituierte Aryloder Aroylreste oder auch alkylierte oder arylierte Organylsilylreste. x, y und z bedeuten dabei Zahlen, die es dem Gesamtmolekül erlaubt, in Wasser löslich oder zumindest dispergierbar zu sein, typischerweise gewählt aus dem Bereich größer als 10, vorteilhaft aus dem Bereich 20 - 10⁷. Im Einzelfalle, beispielsweise wenn der Verdicker aus der Gruppe der derivatisierten Polysaccharide gewählt wird, können die Zahlen x, y bzw. z auch noch wesentlich höhere Werte annehmen. Dies ist dem Fachmanne an sich bekannt und bedarf keiner weiteren Erläuterung.

Für das Strukturschema (2) können beispielsweise folgende spezielleren Strukturschemata befolgt werden: 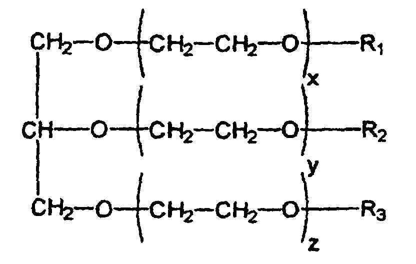 wobei R₁, R₂ und R₃ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 - 10⁷.

Auch Teilsubstitution ist dabei möglich, wobei einer oder mehrere der Indices x, y, oder z den Wert Null annehmen können und einer oder mehrere der Reste R₁, R₂ oder R₃ Wasserstoffatome darstellen können.

Für das Strukturschema (3) können beispielsweise folgende spezielleren Strukturschemata befolgt werden: 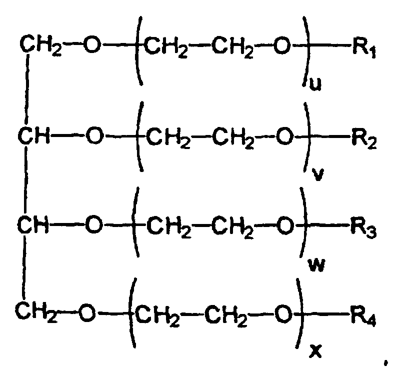 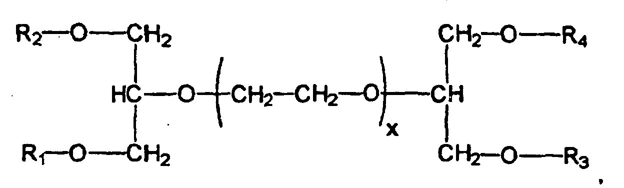 wobei R₁, R₂, R₃ und R₄ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste. u, v, w und x bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 -10⁷.

Auch hier gilt selbstverständlich, daß Teilsubstitution möglich ist, wobei einer oder mehrere der Indices u, v, w, x den Wert Null annehmen können und einer oder mehrere der Reste R₁, R₂ R₃ oder R₄ Wasserstoffatome darstellen können. Dabei gehen die Substanzen natürlich in andere Strukturschemata über.

Für das Strukturschema (9) können beispielsweise folgende spezielleren Strukturschemata befolgt werden: 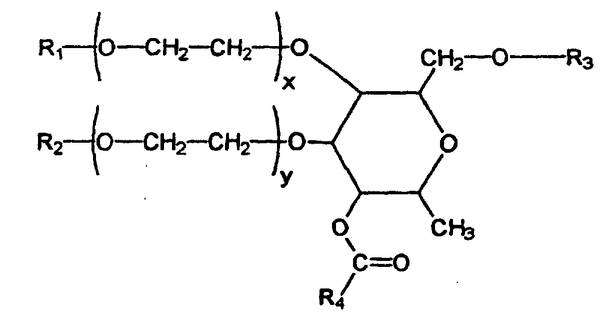 wobei R₁, R₂, R₃ und R₄ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste. x und y bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 - 10⁷.

Für das Strukturschema (10) können beispielsweise folgende spezielleren Strukturschemata befolgt werden: 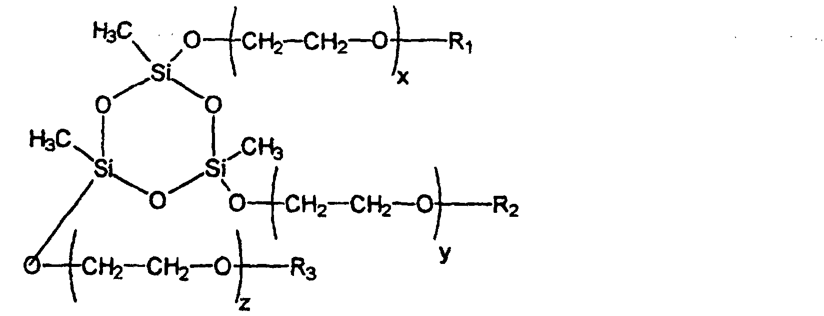 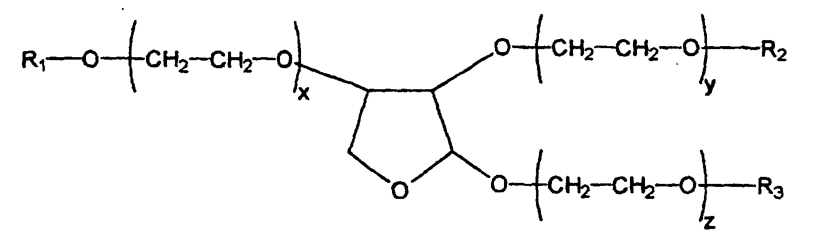 wobei R₁, R₂, und R₃ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 - 10⁷.

Für das Strukturschema (11) kannn beispielsweise folgendes speziellere Strukturschema befolgt werden: 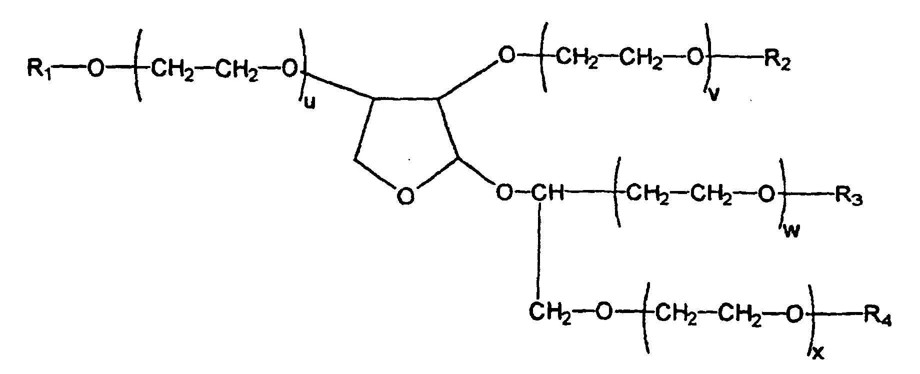 wobei R₁, R₂, R₃ und R₄ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste. u, v, w und x bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 - 10⁷.

Für das Strukturschema (12) kann beispielsweise folgendes speziellere Strukturschema befolgt werden: 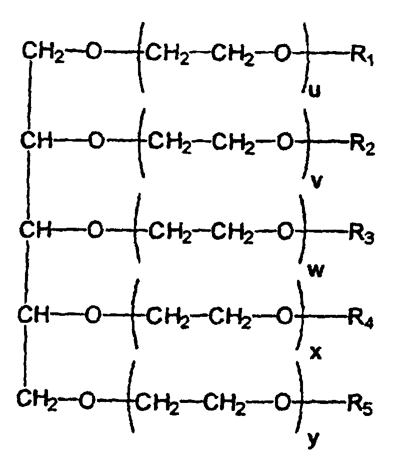 wobei R₁, R₂, R₃, R₄ und R₅ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste, u, v, w, x und y bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 - 10⁷.

Für das Strukturschema (13) kann beispielsweise folgendes speziellere Strukturschema befolgt werden: 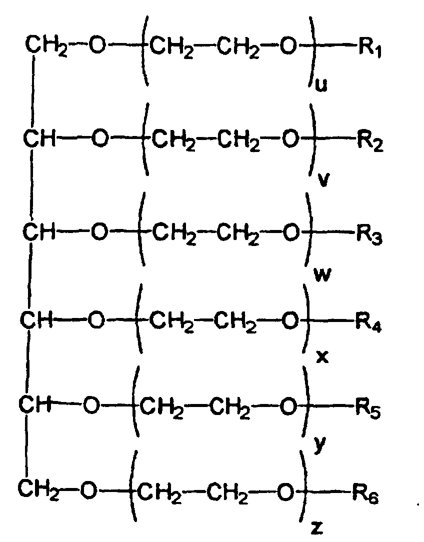 wobei R₁, R₂, R₃, R₄, R₅ und R₆ unabhängig voneinander verzweigte oder unverzweigte, gesättigte oder ungesättigte, cyclische oder kettenförmige aliphatische oder aromatische Reste darstellen können, beispielsweise verzweigte oder unverzweigte oder cyclische Alkyloder Alkanoylreste, mit Alkyl- oder Arylsubstituenten substituierte oder unsubstituierte Aryloder Aroylreste oder auch alkylierte oder arylierte Organylsilylreste. u, v, w, x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 - 10⁷.

Es ist auch von Vorteil, Verdicker zu wählen, welche aus der Gruppe der sogenannten Dendrimere gewählt werden.

Als besonders geeignete Vemetzer haben sich solche herausgestellt, gewählt aus der Gruppe
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl größer als 100 darstellen,
- der veretherten Fettsäureethoxylate der allgemeinen Formel R-COO-(-CH₂-CH₂-O-)ₙ -R', wobei R und R' unabhängig voneinander verzweigte oder unverzweigte Alkyl- oder Alkenylreste und n eine Zahl größer als 100 darstellen,
- der veresterten Fettsäureethoxylate der allgemeinen Formel
   R-COO-(-CH₂-CH₂-O-)ₙ -C(O)-R', wobei R und R' unabhängig voneinander verzweigte oder unverzweigte Alkyl- oder Alkenylreste und n eine Zahl größer als 100 darstellen, der Polypropylenglycolether der allgemeinen Formel
   R-O-(-CH₂-CH(CH₃)-O-)ₙ-R', wobei R und R' unabhängig voneinander verzweigte oder unverzweigte Alkyl- oder Alkenylreste und n eine Zahl größer als 100 darstellen, der veresterten Fettsäurepropoxylate der allgemeinen Formel
   R-COO-(-CH₂CH(CH₃)-O-)ₙ-C(O)-R', wobei R und R' unabhängig voneinander verzweigte oder unverzweigte Alkyl- oder Alkenylreste und n eine Zahl größer als 100 darstellen, der Polypropylenglycolether der allgemeinen Formel
   R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist
- der veretherten Fettsäurepropoxylate der allgemeinen Formel
   R-COO-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen darstellen, deren Summe größer als 100 ist.
- der hydrophob modifizierten wasserlöslichen Polymere der Hydroxyethylcellulose, Polyacrylate (vom Pemulen-Typ), des Polypvinylpyrrolidons, des Polyvinylalkohols, der Glucane, des Pektins, des Polylysins, der Polylglutamate, der Alginate, des Dextrans, der Polymethacrylate, der Copolymere aus Methacrytsäureglercosamid und Cholesterylmethacrylat, der Copolymere aus Polyvinylpyrolidon und Cholesterylmethacrylat, der Metacrylsäureglucosamide.

Insbesondere vorteilhaft ist das PEG-150-Distearat und das PEG-800-Dioleat. Auch das PEG-300-Pentaerythrityltetraisostearat, das PEG-800 Diretinat, das PEG-800 Diglycyrrhetinylstearat und das PEG-800-Ditocopherolat, das PEG-800 Distearat.
Als ganz besonders vorteilhafte Verknüpfersubstanzen haben sich auch die folgende hydrophob modifizierte Polymere herausgestellt: Cetylhydroxyethylcellulose, Stearylhydroxyethylcellulose, Oleylhydroxyethylcelluose, Cholesteryl-polyacrylat, Dodecylamidpolyacrylat, C10-C30 Alkyl Acrylat (Pemulene), Stearylpolyacrylat, Cholesteryldextran, Cholesterylmethacrylat, Methacrylsäure-glucosamid, Copolymer aus Polyvinylpyrolidon und Cholesterylmethacrylat, Stearylpolyvinylalkohol, Copolymer aus Methacrylsäureamid und Cholesterylmethacrylat,

Es kann aber beobachtet werden, daß bei sehr verdünnten Mikroemulsionen, die sich durch einen größeren mittleren Abstand der Öltröpfchen auszeichnen, eine Vernetzung der dispergierten Phase möglich ist, wenn man Polymere mit längeren hydrophilen Gruppen (z.B. längeren Polyethylenoxidketten) den Vorzug gibt. Es ist daher vorteilhaft auch das PEG-800 Distearat verwendet worden.
Ferner können an die Enden des Polymers oder innerhalb des Polymers Gruppen eingefügt werden, die Bestandteil des Hautfetts sind, wie etwa Cholesterin. Durch hauteigene Esterasen oder pH-Wert-Veränderungen können diese Polymere das Cholesterin und den hydrophilen Block nach der Applikation abspalten, sodaß zum Beispiel eine hautpflegende oder hautbefeuchtende Wirkung resultiert. Es ist für den Fachmann naheliegend, daß auch andere Gruppen wie zum Beispiel UV-Filter (wasserlöslich, öllöslich), Antioxidantien, Antiakne-Wirkstoffe, also allgemein bekannte Wirkstoffe aus dem Bereich der Kosmetik, Dermatologie und Pharmazie an wasserlösliche Polymere kovalent gebunden sein können und als solche oder nach Abspaltung der an das wasserlösliche Polymer gebundenen Gruppe die beabsichtigte physiologische Wirkung entfalten.
Bei inneren oder äußeren Wunden können ferner die erfindungsgemäßen Vernetzer nach der Applikation eine immobilisierende oder Kontakt herstellende Funktion (z.B. blutstillend) durch physikalische Vernetzung der Bestandteile von Zellen, Körperflüssigkeiten, oder Körperbestandteilen (Blut, Haar etc.) übernehmen. Die erfindungsgemäßen Vemetzer ermöglichen daher den Verzicht auf eine Wundauflage, Pflaster, Nahtmaterial etc. Polymere auf Basis von Polyethylenglycol sind insbesondere für innere Anwendungen und bei Kontakt mit Blut vorteilhaft, da sie biokompatibel sind, keine immunantwort oder Entzündung hervorrufen. Die Anlagerung von Zellen und Proteinen (Fibrinogen, Immunoglobulin, Leucozyten etc.) wird durch die Hydrophilie, rasche Konformationsänderungen des PEG-Blocks verhindert. Die erfindungsgemäße Modifikation von Polyethylenglycol ist insbesondere vorteilhaft für hydrophobe Gruppen aus körpereigenen Substanzen wie beispielsweise Cholesterin oder auch bioaktive Substanzen, die zum Beispiel ein Antibiotikum oder eine wundheilungsfördende Gruppe freisetzen (Prodrug-Prinzip). Bei einem enzymatischen Abbau der Polymere entstehen hier definierte toxikologisch unbedenkliche Produkte bzw. es werden bioaktive Wirkstoffe freigesetzt. Die Vernetzer erfüllen somit mindestens zwei Aufgaben, da sie einerseits eine niedrigviskose Mikroemulsion (die auch frei von Emulgatoren sein kann) in eine gelartige Darreichungsform überführen und ferner nach der Applikation eine immobilisierende Funktion von Zellen, Körperflüssigkeiten (Blut) oder Körperbestandteilen übernehmen. Beispielsweise vorteilhaft werden bei Wunden oder bei inneren Anwendungen biokompatible Emulgatoren wie Lecithin oder Lecithinderivate mit erfindungsgemäßen Vernetzem eingesetzt.
Die erfindungsgemäßen Vemetzer mit blutstillender Funktion können in Gesichtswasser, Rasierwasser, Pre-shave-Produkte, Aftershave-Lotionen auf Basis einer PIT-Emulsion oder einer Lotion bzw. Creme mit EO-freien Emulgatoren, Rasieröle, schäumende und nicht schäumende Rasiergele, Rasierseifen, Rasierschäume, nachschäumende Rasiergele auf Basis von Mikroemulsionen, erfindungsgemäße Mikroemulsionen, erfindungsgemäße Mikroemulsionsgele, Rasiergele auf Basis von Polyacrylaten, Hydrogelen, Haarentfernungsmitteln, eingebracht werden. Ferner können die Vernetzer oder auch eine Darreichungsform für diese Vernetzer in Vorrichtungen für Rasierklingen eingearbeitet werden.

Vorteilhafte Verknüpfersubstanzen werden beispielsweise gewählt aus der Gruppe mit folgenden Strukturmotiven: 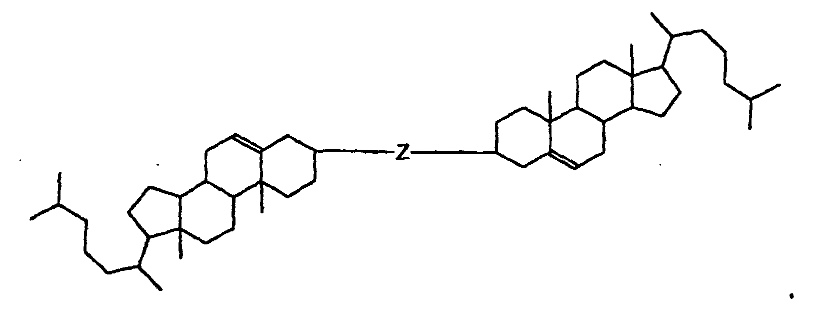 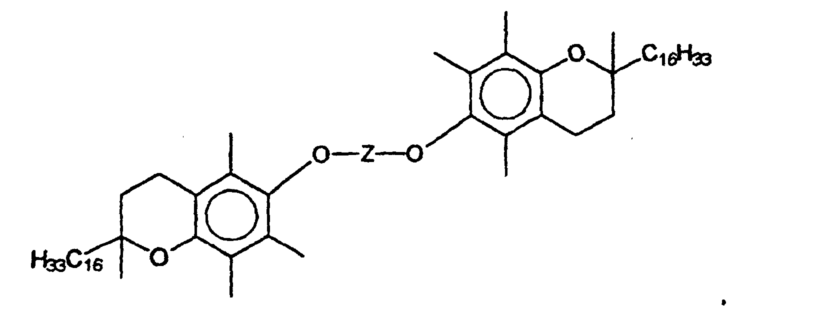 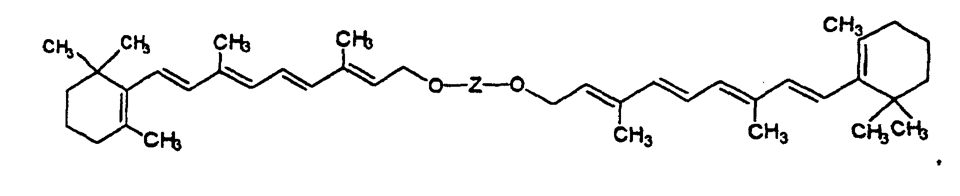 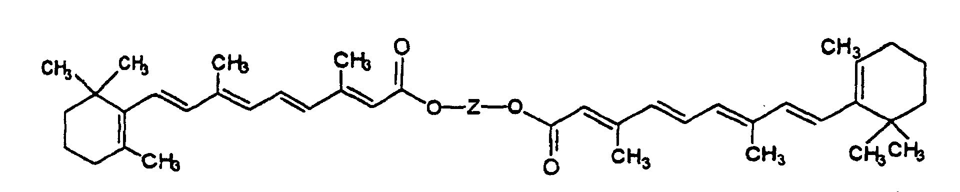 und verwandten Substanzen. Z stellt dabei einen hydrophilen Bereich ein, der besonders vorteilhaft aus der Gruppe der Polyoxyethylengruppen mit Polyethoxylierungsgraden von bis zu 10⁷ gewählt werden kann.

Als besonders vorteilhafte Verknüpfersubstanzen haben sich Dicholesterylverbindungen des Typs 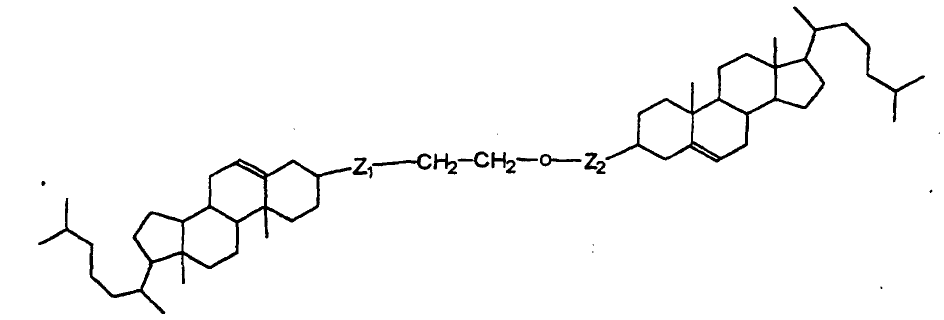 erwiesen, wobei Z₁ und Z₂ unabhängig voneinander gewählt werden können aus der Gruppe Einfachbindung, Estergruppe, Kohlensäureestergruppe, Sauerstoff, Säureamidgruppe, Säureimidgruppe, Thiocarbonsäureestergruppe, Uretahn- bzw. Carbamatgruppe.

Als ganz besonders vorteilhafte Verknüpfersubstanzen haben sich Dicholesterylverbindungen des Typs 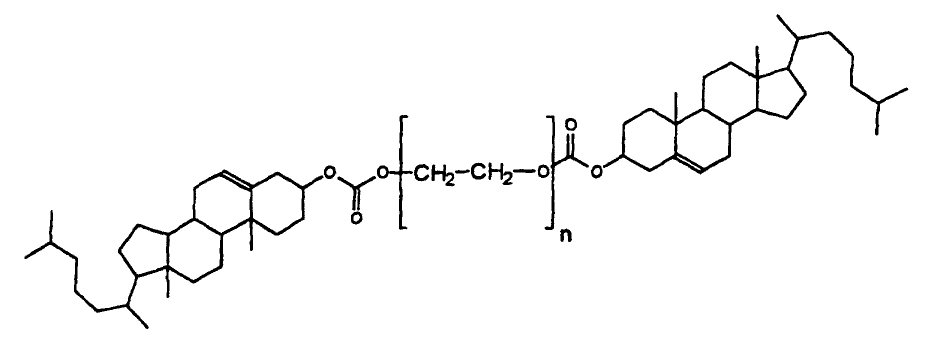 erwiesen, welche wir kollektiv PEG-n-Chol₂ nennen wollen, wobei n Zahlen bedeutet, die es dem Gesamtmolekül erlaubt, in Wasser löslich oder zumindest dispergierbar zu sein, typischerweise gewählt aus dem Bereich größer als 10, vorteilhaft aus dem Bereich 20 bis 10⁷, ganz besonders vorteilhaft aus dem Bereich 120 bis 800.
PEG-n-Chol₂ ist nach den üblichen chemischen Verfahren erhältlich. Insbesondere vorteilhaft kann PEG-n-Chol₂ erhalten werden, indem Polyethylenoxid mit dem gewünschten Polymerisierungsgrade n unter mit einem Cholesterylderivat der allgemeinen Struktur 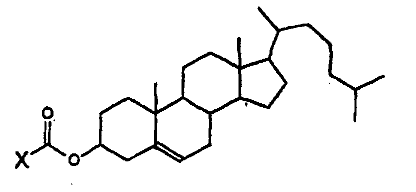 umgesetzt werden, wobei es von Vorteil ist, Reaktionsbedingungen zu schaffen, welche die Abspaltung der Substanz HX begünstigen, etwa nach dem folgenden Reaktionsschema:

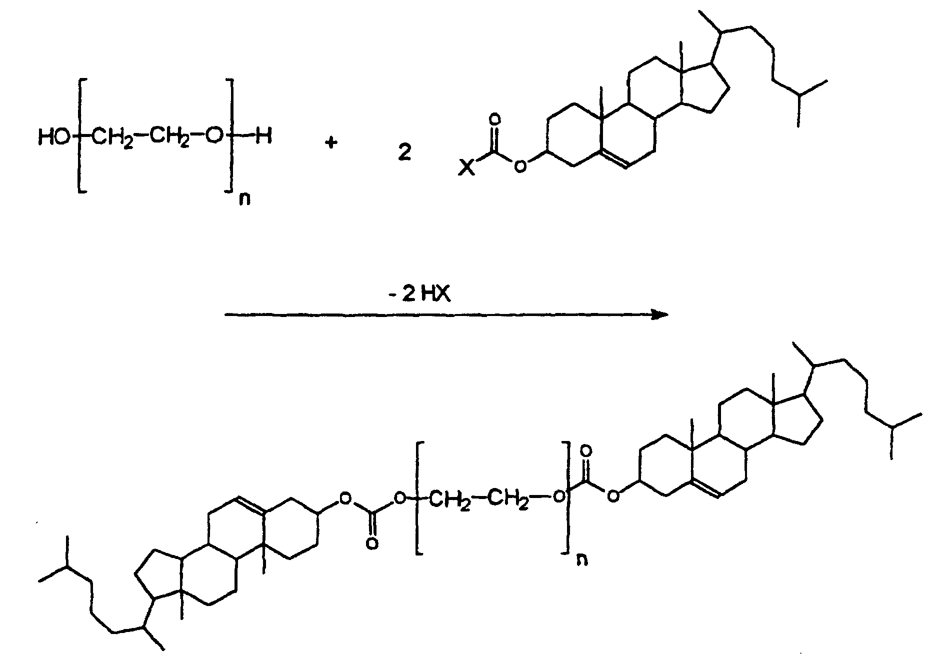

Es ist aber auch vorteilhaft, insbesondere wenn der oder die Verdicker gewählt werden soll aus der Gruppe der assoziativen Verdicker, hydrophob substituierte Polysaccharidderivate zu wählen, beispielsweise der hydrophob substituierten Celluloseether, der hydrophob substituierten Stärken, Alginate, Glucane, Chitine und dergleichen mehr.

Insbesondere vorteilhaft sind die in der US-Patentschrift 5,426,182 beschriebenen hydrophob substituierten Saccharidderivate.

Beispielsweise vorteilhaft kann Cetylhydroxyethylcellulose verwendet werden.

Die Praxis der Herstellung einer erfindungsgemäßen Mikroemulsion besteht demgemäß vorteilhaft darin, nach Auswahl geeigneter Rohstoffe, d.h., Wasser- und Ölphase, ein oder mehrere erfindungsgemäß verwendete ethylenoxidfreie und propylenoxidfreie O/W-Emulgatoren in ein definiertes Mischungsverhältnis zueinander zu setzen und gegebenenfalls weitere Substanzen, besagtes Gemisch zu erhitzen, hernach unter fortwährendem Rühren das Gemisch auf Raumtemperatur abzukühlen.

Zur Herstellung der Mikroemulsionsgele werden z.B. ein oder mehrere der erfindungsgemäß verwendeten Verdicker zu jedem Zeitpunkte der o.a. Herstellung beigefügt.

Besonders vorteilhaft werden aber auch zunächst die niedrigviskosen Mikroemulsionen hergestellt und dazu wird dann der Vernetzer gegeben, der dann die Gelbildung bewirkt.

Die Praxis der Herstellung einer erfindungsgemäßen Mikroemulsion auf Basis von Lecithin oder den vorab beschriebenen Lecithinderivaten besteht demgemäß vorteilhaft darin, ein oder mehrere erfindungsgemäß verwendete ethylenoxidfreie und propylenoxidfreie O/W-Emulgatoren, Lecithin und eine oder mehrere Ölphasen in ein definiertes Mischungsverhältnis zueinander zu setzen und gegebenenfalls weitere Substanzen, besagtes Gemisch langsam mit Wasser zu versetzen, so daß ein Gel entsteht, daß bei weiterer Wasserzugabe und gegebenenfalls der Zugabe weiterer Substanzen zu den erfindungsgemäßen Mikroemulsionen führt.
Zur Herstellung der Mikroemulsionsgele werden eine oder mehrere der erfindungsgemäß verwendeten Verdicker zu jedem Zeitpunkte der o.a. Herstellung beigefügt.

Insbesondere vorteilhaft im Sinne der vorliegenden Erfindung sind Mikroemulsionsgele,
(a) beruhend auf Mikroemulsionen vom Typ Öl-in-Wasser, welche umfassen
   - eine diskontiniuerliche Ölphase und eine kontinuierliche Wasserphase
   - enthaltend mindestens einen ethylenoxidfreien und propylenoxidfreien O/W-Emulgator
   - wobei dieser ausgewählt wird aus der Gruppe der Acyl-Lactylate, Glutamate, Sarcosinate, Isethionate, Sulfosucinate, Alaninate, Amphoacetate, Polyglycerinester, Alkylglycoside, Alkylpolyglycoside, Sorbitanester, Methylglucoseester, Ester von Hydroxysäuren und der Polyglycerinmethylglucoseester,
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

Die Menge der erfindungsgemäßen Verdicker sollte vorzugsweise im Bereich von 0,3 bis 30 Gew.-%, insbesondere 1 bis 10 Gew.-% liegen, jeweils bezogen auf das Gesamtgewicht des Mikroemulsionsgeles.

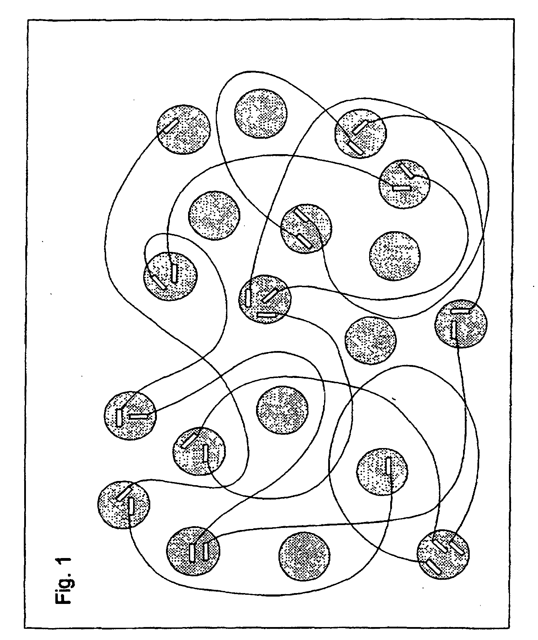

In Fig. 1 wird das erfindungsgemäße Prinzip verdeutlicht: Die als schraffierte Kreise dargestellt Mikroemulsionströpfchen einer O/W-Mikroernulsion werden durch die als Linien dargestellten Vernetzermoleküle miteinander verbunden, welche an beiden Enden durch Rechtecke symbolisierte hydrophobe Reste tragen. Ersichtlich ist, daß ein Emulsionströpfchen grundsätzlich auch mehrere hydrophobe Reste beherbergen kann, wodurch eine stärkere Vernetzung und Dreidimensionalität des Netzwerks gewährleistbar ist.

Eine zweite erfindungsgemäße Möglichkeit der Bildung von Mikroemulsionsgelen besteht darin, die Öltröpfchen durch den Einsatz hydrophob modifizierter, synthetischer oder natürlicher wasserlöslicher/dispergierbarer Polymere zu immobilisieren. Solche Polymere werden auch als assoziative Verdicker bezeichnet.

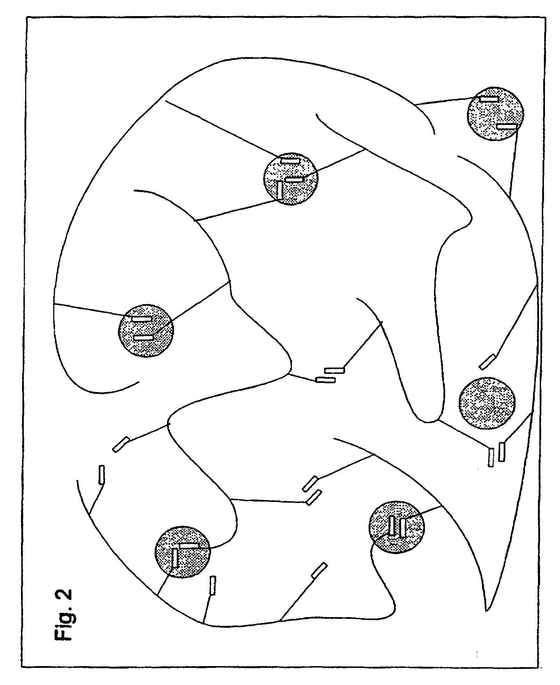

In Fig. 2 wird dieses Prinzip verdeutlicht. Das durch einen in Fig. 2 nicht bezeichneten Wasseranteil gequollene Gelgerüst wird im wesentlichen durch die als verzweigte Linien dargestellten Vernetzermoleküle aufgebaut, welche an den Enden der Verzweigungen durch Rechtecke symbolisierte hydrophobe Reste tragen. Die hydrophoben Reste lagern sich durch hydrophobe Wechselwirkung aneinander, wodurch Vernetzung bewirkt wird. An den Vemetzungsstellen können Mikroemulsionströpfchen durch hydrophobe Wechselwirkung ebenfalls anlagern. Es ist dabei für wohl grundsetzlich unerheblich, ob die hydrophoben Reste "eintauchen" oder ob die hydrophoben Reste lediglich oberflächlich mit den Mikroemulsionströpfchen in Kontakt treten und mehr oder weniger stark an dieser haften.

Insbesondere ist vorteilhaft, wenn der/die ethylenoxidfreie/n und propylenoxidfreie/n O/W-Emulgator/en gewählt wird oder werden aus der Gruppe der
- Acyllactylate der Formel
   R-C(O)O-CH(CH₃)-C(O)O-CH(CH₃)CO₂⁻M⁺, wobei R eine gesättigte und/oder ungesättigte, verzweigte und/oder unverzweigte Fettsäure mit 6 bis 26 C-Atomen darstellt.
- der Acylglutamate der Formel
   R-C(O)NHCH(COO⁻, M⁺)CH₂CH₂COO⁻ M⁺, wobei R eine gesättigte und/oder ungesättigte, verzweigte und/oder unverzweigte Fettsäure mit 6 bis 26 C-Atomen darstellt.
- der Acylsarcosinate der Formel
   R-C(O)-N(CH₃)CH₂COO⁻ M⁺, wobei R eine gesättigte und/oder ungesättigte, verzweigte und/oder unverzweigte Fettsäure mit 6 bis 26 C-Atomen darstellt.
- der isethionate der Formel
   RC(O)-O-CH₂CH₂-SO₃⁻M⁺, wobei R eine gesättigte und/oder ungesättigte, verzweigte und/oder unverzweigte Fettsäure mit 6 bis 26 C-Atomen darstellt.
- der Sulfosucinate der Formel
   M⁺,⁻O-C(O)-CH₂-CH(SO₃-M+)-C(O)-O-R, wobei R eine gesättigte und/oder ungesättigte, verzweigte und/oder unverzweigte Fettsäure mit 6 bis 26 C-Atomen darstellt.
- der Alaninate der Formel

   CH₃CH₂N(CH₃)(C₁₂H₂₅)C(O)O⁻ M⁺
- der Amphoacetate der Formel

   R-C(O)-NH-CH₂CH₂-N(CH₂CH₂OH)-CH₂COO⁻; M⁺
- der Polyglycerinester, Alkylglycoside, Alkylpolyglycoside, Sorbitanester, Sucroseester, vorzugsweise Sucroselaurat, insbesondere Sucrosemonolaurat (z.B. Sistema L70-C), Methylglucoseester, Ester von Hydroxysäuren und der Polyglycerinmethylglucoseester.

Es ist von besonderem Vorteil, als Acyl Lactylat das Natrium Lauroyl Lactylat und das Natrium Caproyl Lactylat zu verwenden.
Ferner hat sich als Glutamat das Natrium Lauroylglutamat und das Natriumcocoylglutamat bewährt.
Vorteilhaft täßt sich auch Natrium Lauroylsarcosinat verwenden.
Es ist von besonderem Vorteil als Isethionat das Natrium Lauroylisethionat zu verwenden.
Femer erwies sich das Dinatriumlauryl Sulfosuccinat als geeignet.
Als Alaninat erwieß sich das N-Methyl-N-Iauroyl-Alaninat als geeignet.
Als Amphoacetat war Natriumtauroamphoacetate gut geeignet.
Vorteilhaft war auch als Polyglycerinester das Polyglycerinester-laurat, Polyglycerin-10 Monooleat, Polyglycerin-10 Monoisostearat, , Polyglycerin-10 Monostearat geeignet.
Als Alkylpolyglycosid war Laurylglycosid geeignet.Es ist ebenfalls günstig, als Sorbitanester das Sorbitanstearat zu verwenden. Als Ester von Hydroxysäuren haben sich C12-13 Alkyl Apfelsäureester und C12-13 Weinsäureester bewährt.
Als fakultative, dennoch erfindungsgemäß vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen, Methylglucoseester gesättigter und/oder ungesättigter, verzweigter und/oder unverzweigter Alkancarbonsäuren einer Kettenlänge von 8 bis 24, insbesondere 12 - 18 C-Atomen. Geeignete Lecithinderivate sind z.B. hydrierte, teilhydrierte und nicht hydrierten Phospholipide, Phosphatidylcholin, Phosphatidylethanolamin, Phosphatidylserin, Cardiolipin (Diphosphatidylglycerin) und Sphingomyelin, Ceramide.
Insbesondere vorteilhafte W/O-Emulgatoren sind Glyerinoleat, Glycerylmonostearat, Glycerylmonoisostearat, Diglycerylmonostearat, Diglycerylmonoisostearat, Diglycerindiisostearat , Polygycerin-3 Disostearat, Natriumisostearyllactylat, Propylenglycolmonostearat, Sorbitanmonoisostearat, Cetylalkohol, Arachidylalkohol, Selachylalkohol, Chimylalkohol, Glycerylmonolaurat, Glycerylmonocaprinat, Glycerylmonocaprylat, hydriertes und nicht hydriertes Lecithin, Methylglucose Isostearat, 2-Ethylhexylglycerinether, Methylglucose Distearat, Laurylglycol, Lauryllactat.
Auch kommerziell erhältliche Nanoemulsionen oder Mikroemulsionen zum Beispiel von den Firmen Kuhs beziehungsweise Nattermann ("Probiol"), Gattefossé, Rovi, Vesifact AG sind vorteilhaft zu verwenden und können gewünschtenfalls mit Wirkstoffen, z.B. Hautbefeuchtungsmitteln, Vitamin C, SOD, UV-Filtern, Plasmid DNA, epidermale Wachstumsfaktoren (EGF, FGF, PDGF), Glyocosylrutin, Q10, Cyclip AMP, Tyrosin, Amphotericin B, Daunorubicin, Ibuprofen, Doxorubicin, Cyclosporin, T4 Endonuclease, und dergleichen beladen werden, aber auch als unbeladene Nanoemulsionen oder Mikroemulsionen zu erfindungsgemäßen Gelen führen. Der Fachmann weiß, daß es weitere Firmen gibt, die beladene oder unbeladene Nanoemulsionen oder Mikroemulsionen anbieten, die nach dem hier vorgestelltem Verfahren zu erfindungsgemäßen Gelen führen. So lassen sich zum Beispiel durch Hochdruckhomogeniserung emulgatorfreie oder emulgatorarme Nanoemulsionen und Mikroemulsionen herstellen. Das erfindungsgemäße Prinzip der Vernetzung führt auch hier zu gelartigen Zubereitungen.
Ferner ist bekannt, daß bei Liposomenzubereitungen mit hohem Ölanteil Nanoemulsionen erhalten werden können oder auch Liposomen und Nanoemulsionen nebeneinander vorliegen können. Das erfindungsgemäße Prinzip der Vernetzung führt auch hier zu Gelen, da die Verankerung der hydrophoben Enden des wasserlöslichen Polymers dann in den Bilayermembranen der Vesikel und in den Mikroemulsionströpfen stattfinden kann.

Es ist erfindungsgemäß auch möglich, den Gesamtgehalt an Emulgatoren kleiner als 15 Gew.-%, bezogen auf das Gesamtgewicht der erfindungsgemäßen Mikroemulsionen, zu halten. Es wird bevorzugt, den Gesamtgehalt an Emulgatoren kleiner als, 10 Gew-.%, insbesondere kleiner als 8 Gew.-%, bezogen auf das Gesamtgewicht zu halten.
Insbesondere kann der Gesamtgehalt an Emulgatoren z.B. 0.1 bis 20 Gew.-% betragen, bezogen auf das Gesamtgewicht der Mikroemulsion.

Die Ölphase der erfindungsgemäßen Mikroemulsionsgele wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.
Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Es kann auch gegebenenfalls vorteilhaft sein, Wachse, beispielsweise Cetylpalmitat, als alleinige Lipidkomponente der Ölphase einzusetzen. In solchen Fällen können die erfindungsgemäßen O/W-Mikroemulsionen auch gegebenenfalls als Mikrodispersionen fester Wachspartikel anfallen. '

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Der Anteil der Ölphase kann z.B. 0,01 bis 30 Gew.-% betragen, bezogen auf das Gesamtgewicht der Mikroemulsionen.

Der Anteil der Wasserphase kann stark variieren und z.B. 1 bis 99 Gew.-% betragen.

Der Anteil der O/W-Emulgatoren kann z.B. 0,01 bis 20 Gew.-% betragen, bezogen auf das Gesamtgewicht der Mikroemulsion.

Gewünschtenfalls kann der Anteil der W/O-Emulgatoren z.B. 0,01 bis 15 Gew.-% betragen, bezogen auf das Gesamtgewicht der Mikroemulsion.

Erfindungsgemäß können vorteilhafte O/W-Mikroemulsionen und -gele erhalten werden, wobei der Anteil des O/W-Emulgators z.B. unter 20 Gew.-%, insbesondere z.B. unter 15 Gew.-%, bezogen auf das Gesamtgewicht der Mikroemulsion liegt, und z.B. weniger als 15 Gew.-%, insbesondere weniger als 5 Gew.-%, eines zusätztichen W/O-Emulgators vorliegen, wobei der oder die erfindungsgemäß verwendetenen Verdicker zu jedem Zeitpunkte der Herstellung beigefügt werden können.

Es ist dabei im Einzelfalle möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Die erfindungsgemäßen Mikroemulsionsgele enthalten vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Die erfindungsgemäßen Mikroemulsionsgele tragen ferner in vorzüglicher Weise zur Hautglättung bei, insbesondere, wenn sie mit einer oder mehreren Substanzen versehen sind, die die Hautglättung fördern.

Stellen die erfindungsgemäßen Mikroemulsionsgele Grundlagen für kosmetische Desodorantien/Antitranspirantien dar, so können alle gängigen Wirkstoffe vorteilhaft genutzt werden, beispielsweise Geruchsüberdecker wie die gängigen Parfümbestendteile, Geruchsabsorber, beispielsweise die in der Patentoffenlegungsschrift DE-P 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Mikroemulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11.Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Die üblichen Antitranspiranswirkstoffe können ebenfalls vorteilhaft in den erfindungsgemäßen Mikroemulsionsgele verwendet werden, insbesondere Adstringentien, beispielsweise basische Aluminiumchloride.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren Mikroemulsionsgelen.

Als Treibmittel für erfindungsgemäße, aus Aerosolbehältem versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Es hat sich darüberhinaus in überraschender Weise herausgestellt, daß bei der Verwendung von in der Ölphase löslichen Treibmitteln, also beispielsweise üblichen Propan-Butan-Gemischen, die erfindungsgemäßen O/W-Mikroemulsionsgele nicht einfach als Aerosoltröpfchen versprüht werden, sondern sich zu feinblasigen, reichhaltigen Schäumen entwikkeln, sobald solche mit solchen Treibmitteln beladenen Systeme Druckentspannung erfahren.

Solche nachschäumenden Zubereitungen werden daher ebenfalls als vorteilhafte Verkörperungen der vorliegenden Erfindung mit eigenständiger erfinderischer Tätigkeit angesehen.

Bei der Verwendung von in der Ölphase unlöslichen Treibmitteln werden die erfindungsgemäßen O/W-Mikroemulsionsgele als Aerosoltröpfchen versprüht.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethythexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicytsäure(4-isopropylbeuzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden. Es ist dabei vorteilhaft. Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen Mikroemulsionsgele mit einem Gehalt an einem oder mehreren Antioxidantien zu versehen, wenn die Zubereitungen einem anderen Zwecke dienen sollen, z.B. als Desodorantien oder Sonnenschutzmittel.

### Besonders vorteilhaft werden die Antioxidantien gewählt aus der Gruppe bestehend aus

Aminosäuren (z.B. Histidin, Tyrosin, Tryptophan) und deren Derivate, Imidazole (z.B. Urocaninsäure) und deren Derivate, Peptide wie D,L-Camosin, D-Camosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptahioninsulfoximin) in sehr geringen verträglichen Dosierungen (z.B. pmol bis µmol/kg), femer (Metall)-Chelatoren (z.B. α-Hydroxyfettsäuren, α-Hydroxypalmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate. Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Zink und dessen Derivate (z.B. ZnO, ZnSO₄) Seten und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw. Der Gewichtsanteil an Hilfs- oder Zusatzstoffen kann z.B. 0,001 bis 20 Gew.-% betragen, bezogen auf das Gesamtgewicht der Mikroemulsion.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramid-ähnliche Verbindungen und so weiter.

Obgleich selbverständlich auch die Verwendung hydrophiler Wirkstoffe erfindungsgemäß begünstigt ist, ist ein weiterer Vorteil der erfindungsgemäßen Mikroemulsionsgele, daß die hohe Anzahl feinstzerteilter Tröpfchen gerade öllösliche bzw. lipophile Wirkstoffe mit besonders großer Wirksamkeit biologisch verfügbar macht.

Vorteilhaft ist es auch, die Wirkstoffe aus der Gruppe der rückfettenden Substanzen zu wählen, beispielsweise Purcellinöl, Eucerit® und Neocerit®. Das Gewichtsanteil der Wirkstoffe kann z.B. 0.001 bis 20 Gew.-% betragen, bezogen auf das Gesamtgewicht der Mikroemulsion.

Es ist auch möglich und gegebenenfalls vorteilhaft, den erfindungsgemäßen Zubereitungen waschaktive Tenside zuzufügen. Erfindungsgemäße wäßrige kosmetische Reinigungsmittel oder für die wäßrige Reinigung bestimmte wasserarme oder wasserfreie Reinigungsmittelkonzentrate können kationische, anionische, nichtionische und/oder amphotere Tenside enthalten, beispielsweiseherkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaïne, Sulfosuccinate, Sulfobernsteinsäurehalbester, Alkylethercarboxylate, Eiweiß-Fettsäure-Kondensate, Alkylbetaïne und Amidobetaïne, Fettsäurealkanolamide, Polyglycolether-Derivate.

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, mindestens einen erfindungsgemäßes Elektrolyten und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, gegebenenfalls Elektrolyte und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen. Vorteilhaft sind beispielsweise Cetyltrimethylammoniumsalze zu verwenden.

Die erfindungsgemäßen für die Reinigung des Haares oder der Haut vorgesehenen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die erfindungsgemäßen Zubereitungen haben, trotz ihres Ölgehaltes, in erstaunlicher Weise sehr gute Schaumentwicklung, hohe Reinigungskraft und wirken in hohem Maße regenerierend in bezug auf den allgemeinen Hautzustand. Insbesondere wirken die erfindungsgemäßen Zubereitungen hautglättend, vermindern das Trockenheitsgefühl der Haut und machen die Haut geschmeidig.

Sollen die erfindungsgemäßen Mikroemulsionsgele zur Haarpflege eingesetzt werden, können sie die üblichen Bestandteile enthalten, üblicherweise zum Beispiel filmbildende Polymere. Von solchen Polymeren mit wenigstens teilweise quaternisierten Stiekstoffgruppen (im folgenden "Filmbildner" genannt), eigenen sich bevorzugt solche, welche gewählt werden aus der Gruppe der Substanzen, welche nach der INCI-Nomenklatur (International Nomenclature Cosmetic Ingredient) den Namen "Polyquaternium" tragen, beispielsweise:
- Polyquaternium-2: (Chemical Abstracts-Nr. 63451-27-4, z.B. Mirapol® A-15)
- Polyquaternium-5: (Copolymeres aus dem Acrylamid und dem β-Methacryloxyethyltrimethylammoniummethosulfat, CAS-Nr. 26006-22-4)
- Polyquaternium-6: (Homopolymer des N,N-Dimethyl-N-2-propenyl-2-propen-1-aminiumchlorids, CAS-Nr. 26062-79-3, z.B. Merquat® 100
- Polyquaternium-7: N,N-Dimethyl-N-2-propenyl-2-propen-1-aminiumchlorid, Polymeres mit 2-Propenamid, CAS-Nr. 26590-05-6, z.B. Merquat® S
- Polyquaternium-10: Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M,
- Polyquaternium-11: Vinylpyrrolidon/dimethylaminoethyl-Methacrylat-Copolymer/Diethylsulfat-Reaktionsprodukt, CAS-Nr. 53633-54-8, z.B. Gafquat® 755N
- Polyquaternium-16: Vinylpyrrolidon/vinylimidazoliniummethochlorid-Copolymer, CAS-Nr. 29297-55-0, z.B. Luviquat® HM 552
- Polyquaternium-17: CAS-Nr. 90624-75-2, z.B. Mirapol® AD-1
- Polyquaternium-19: Quaternisierter wasserlöslicher Polyvinylalkohol
- Polyquaternium-20: in Wasser dispergierbarer quaternisierter Polyvinyloctadecylether
- Polyquaternium-21: Polysiloxan-polydimethyl-dimethylammoniumacetat-Copolymeres, z.B. Abil® B 9905
- Polyquaternium-22: Dimethyldiallylammoniumchlorid/Acrylsäure-Copolymer, CAS-Nr. 53694-7-0, z.B. Merquat® 280
- Polyquaternium-24: Polymeres quaternäres Ammoniumsalz der Hydroxyethylcellulose, Reaktionsprodukt mit einem mit Lauryldimethylammonium substituierten Epoxid, CAS-Nr. 107987-23-5, z.B. Quatrisoft® LM-200
- Polyquaternium-28: Vinylpyrrolidon/Methacrylamidopropyltrimethylammoniumchlorid-Copolymer, z.B. Gafquat® HS-100
- Polyquaternium-29: z.B. Lexquat® CH
- Polyquaternium-31: CAS-Nr. 136505-02-7, z.B. Hypan® QT 100
- Polyquaternium-32: N,N,N-trimethyl-2-[(2-methyl-1-oxo-2-propenyl)oxy]-Ethanaminiumchlorid, polymer mit 2-Propanamid, CAS-Nr. 35429-19-7
- Polyquaternium-37: CAS-Nr. 26161-33-1

Vorteilhaft enthalten erfindungsgemäße Zubereitungen zur Haarpflege 0.2 - 50 Gew.-% eines oder mehrerer Filmbildner, bevorzugt 5 - 30 Gew.-%, insbesondere 10 - 25 Gew.-%, jeweils bezogen auf das Gesamtgewicht der Zubereitungen. Derartige Ausführungsformen der erfindungsgemäßen Zubereitungen pflegen durch Umwelteinflüsse geschädigtes oder strapaziertes Haar bzw. beugen solchen Umwelteinflüssen vor. Ferner verleihen die erfindungsgemäßen Zubereitungen der Haartracht lockere Fülle und Festigkeit, ohne klebrig zu wirken.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeignet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens, Farbstoffe, Pigmente, die färbende Wirkung haben, weitere, nicht unter die Definition der erfindungsgemäßen Verdicker fallende Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Insbesondere vorteilhaft werden Gemische der vorstehend genannten Lösungsmittel verwendet.

Als weitere Bestandteile können verwendet werden Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte.

Alle gestellten Aufgaben werden gemäß der Erfindung gelöst.

Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die nachfolgenden Beispiele sollen die vorliegende Erfindung verdeutlichen.

Die nachfolgenden Mengenangaben beziehen sich auf das Gewicht oder sind Gew.-%-Angaben.

### Herstellungsbeispiel für PEG-140-Chol₂

36 g (6 mmol) Polyethylenoxid (M = 6.000 gmol⁻¹, n ≈ 140) werden in 50 ml Benzol gelöst und durch Gefriertrocknung von enthaltenen Wasserspuren befreit. Anschließend wird das Polyethylenoxid in 70 ml frisch absolutiertem Dichlormethan aufgenommen. Unter Stickstoffatmosphäre werden 10,8 g (24 mmol) Cholesterylchloroformiat und 5 ml Pyridin (letzteres über CaH₂ destilliert) zugesetzt. Das Polymer wird in 1,5 l Diethylether ausgefällt und durch wiederholtes Umfällen (dreimal) aus Dichlormethan/ Diethylether gereinigt. Das vorgetrocknete Produkt wird in ca 1 I warmen Acetons gelöst und fällt bei 0° C quantitativ aus.

### Herstellungsbeispiel für PEG-180-Chol₂

48 g (6 mmol) Polyethylenoxid (M = 8.000 gmol⁻¹, n ≈ 180) werden in 70 ml Benzol gelöst und durch Gefriertrocknung von enthaltenen Wasserspuren befreit. Anschließend wird das Polyethylenoxid in 100 ml frisch absolutiertem Dichlormethan aufgenommen. Unter Stickstoffatmosphäre werden 10,8 g (24 mmol) Cholesterylchloroformiat und 5 ml Pyridin (letzteres über CaH₂ destilliert) zugesetzt. Das Polymer wird in 1,5 I Diethylether ausgefällt und durch wiederholtes Umfällen (dreimal) aus Dichlormethan /Diethylether gereinigt. Das vorgetrocknete Produkt wird in ca 1 I warmen Acetons gelöst und fällt bei 0° C quantitativ aus.

Ausbeute: 44 g PEG-180-Chol₂ (4,8 mmol), entsprechend 80 % der Theorie.

### Herstellungsbeispiel für PEG-450-Chol₂

120 g (6 mmol) Polyethylenoxid (M = 20.000 gmol⁻¹, n ≈ 450) werden in 170 ml Benzol gelöst und durch Gefriertrocknung von enthaltenen Wasserspuren befreit. Anschließend wird das Polyethylenoxid in 100 ml frisch absolutiertem Dichlormethan aufgenommen. Unter Stickstoffatmosphäre werden 10,8 g (24 mmol) Cholesterylchloroformiat und 5 ml Pyridin (letzteres über CaH₂ destilliert) zugesetzt. Das Polymer wird in 2,5 I Diethylether ausgefällt und durch wiederholtes Umfällen (dreimal) aus Dichlormethan /Diethylether gereinigt. Das vorgetrocknete Produkt wird in ca 1 I warmen Acetons gelöst und fällt bei 0° C quantitativ aus.

### Herstellungsbeispiel für PEG-800-Chol₂

58 g (6 mmol) Polyethylenoxid (M = 35.000 gmol⁻¹, n ≈ 800) werden in 130 ml Benzol gelöst und durch Gefriertrocknung von enthaltenen Wasserspuren befreit. Anschließend wird das Polyethylenoxid in 100 ml frisch absolutiertem Dichlormethan aufgenommen. Unter Stickstoffatmosphäre werden 10.8 g (24 mmol) Cholesterylchloroformiat und 5 ml Pyridin (letzteres über CaH₂ destilliert) zugesetzt. Das Polymer wird in 1,5 I Diethylether ausgefällt und durch wiederholtes Umfällen (dreimal) aus Dichlormethan /Diethylether gereinigt. Das vorgetrocknete Produkt wird in ca 1 I warmen Acetons gelöst und fällt bei 0° C quantitativ aus.

### Herstellungsbeispiel für Cholesteryl-N-(6-isocyanatohexyl)carbamal (Substanz 1)

7,8 g Cholesterol werden mit 48 ml 1,6-Hexyldiisocyanat in 200ml absolutem Toluol gelöst. Nach Zugabe von 4 ml Pyridin wird die Lösung 48h bei 80° C gehalten. Das Lösungsmittel wird anschließend vollständig abdestilliert und der Rückstand in 600 ml Petrolether (Siedebereich 40-60° C) aufgenommen. Bei -10° C fällt das Produkt aus. Der Niederschlag wird abgesaugt, mit weiterem Petrolether gewaschen und anschließend im Ölpumpenvakuum getrocknet.

### Herstellungsbeispiel für Cholesterylpolyacrylat

5 g Polyacrylsäure (M = 450.000) und 5 ml Pyridin werden in 150 ml wasserfreiem N-Methylpyrrolidon bei 60° C gelöst. Dann tropft man eine Lösung von 0,555 g ( 1 mmol) von Substanz 1 in 10 ml N-Methylpyrrolidon zu. Der Reaktionsansatz wird 24 h bei 60° C gerührt und dann mit Aceton ausgefällt. Die ausgefallene Substanz wird in ca. 100 ml Wasser gegeben und mit 20- 40ml Natronlauge (40 %-ig) versetzt. Man behandelt das Gel mehrmals mit Aceton und trocknet dann bei 10 mbar. Die erhaltene Masse wird in ca. 250 ml Wasser gelöst, mit Methanol ausgefällt und im Membranpumpenvakuum getrocknet. Man wiederholt diesen Vorgang und trocknet dann 24 h bei einem Druck von 10⁻² mbar.

### Herstellungsbeispiel für Cholesteryldextran

3 g Dextran und 0,2g 1 werden in Gegenwart von 5 ml Pyridin in 80ml DMSO umgesetzt (8h bei 80° C). Nach Zugabe von 500 ml Ethanol fällt das Produkt bei 0° C aus. Die weitere Reinigung erfolgt durch Dialyse gegen Wasser.

### Herstellungsbeispiel für Cholesterylhydroxyethylcelluose

Hydroxyethylcellulose wird 24 h bei 60° C und einem Druck von 10⁻² mbar getrocknet. Eine Mischung von 2 g getrockneter Hydroxyethylcellulose, 120 ml wasserfreiem N-Methylpyrrolidon und 30 ml wasserfreiem Pyridin wird entgast und unter Argon 38 h bei 60° C gerührt. Die leicht gelb gefärbte, hochviskose Lösung wird mit 0.09 g (0.20 mmol) Cholesterinchloroformiat in 7 ml N-Metylpyrrolidon versetzt. Man rührt 18 h bei 60° C unter Argon. Die Cholesterylhydroxyethylcelluose wird in Aceton gefällt und bei 10 mbar getrocknet. Zur weiteren Reinigung wird die Cholesterylhydroxyethylcelluose 24 h im Soxhlet-Extraktor mit Benzol extrahiert und anschließend 24 h bei 10⁻² mbar getrocknet.

### Herstellungsbeispiel für Stearyllhyroxyethylcellulose

Hydroxyethylcellulose wird 24 h bei 60° C und einem Druck von 10⁻² mbar getrocknet. Eine Mischung von 2 g getrockneter Hydroxyethylcellulose,120 ml wasserfreiem N-Methylpyrrolidon und 30 ml wasserfreiem Pyridin wird entgast und unter Argon 38 h bei 60 °C gerührt. Die leicht gelb gefärbte, hochviskose Lösung wird mit 0,06 g (0,20 mmol) Stearinsäurechlorid in 5 ml N-Methylpyrrolidon versetzt. Man rührt 24 h bei 60° C unter Argon. Die Stearylhydroxyethylcellulose wird in Aceton gefällt und bei 10 mbar getrocknet. Man löst die Stearyllhydroxyethylcellulose in ca. 200 ml Wasser (24 h rühren), fällt erneut aus Aceton und trocknet 24 h bei 10⁻² mbar.

### Herstellungsbeispiel für Oleylhydroxyethylcellulose

Hydroxyethylcellulose wird 24 h bei 60° C und einem Druck von 10⁻² mbar getrocknet. Eine Mischung von 2 g getrockneter Hydroxyethylcellulose, 120 ml wasserfreiem N-Methylpyrrolidon und 30 ml wasserfreiem Pyridin wird entgast und unter Argon 38 h bei 60° C gerührt. Die leicht gelb gefärbte, hochviskose Lösung wird mit 0,06 g (0,20 mmol) Ölsäurechlorid in 5 ml N-Methylpyrrolidon versetzt. Man rührt 24 h bei 60° C unter Argon. Die Oleylhydroxyethylcellulose wird in Aceton gefällt und bei 10 mbar getrocknet. Man löst die Oleylhydroxyethylcellulose in ca. 200 ml Wasser (24 h rühren), fällt erneut aus Aceton und trocknet 24 h bei 10⁻² mbar.

### Herstellungsbeispiel für Palmitylhdroxyethylcellulose

Hydroxyethylcellulose wird 24 h bei 60° C und einem Druck von 10⁻² mbar getrocknet. Eine Mischung von 2 g getrockneter Hydroxyethylcellulose, 120 ml wasserfreiem N-Methylpyrrolidon und 30 ml wasserfreiem Pyridin wird entgast und unter Argon 38 h bei 60 oC gerührt. Die leicht gelb gefärbte, hochviskose Lösung wird mit 0,05 g (0,20 mmol) Palmitinsäurechlorid in 5 ml NMetylpyrrolidon versetzt. Man rührt 24 h bei 60 oC unter Argon. Die Palmitylhdroxyethylcellulose wird in Aceton gefällt und bei 10 mbar getrocknet. Man löst die Palmitylhdroxyethylcellulose in ca. 200 ml Wasser (24 h rühren), fällt erneut aus Aceton und trocknet 24 h bei 10⁻² mbar.

### Herstellungsbeispiel für Dodecylpolyacrylat

5 g Polyacrylsäure (M = 450.000) und eine Spatelspitze 4-Dimethylaminopyridin werden in 150 ml wasserfreiem N-Methylpyrrolidon bei 60° C gelöst. Dann tropft man eine Lösung von 0,389 g (2,10 mmol) Dodecylamin und 0,475 g (2,30 mmol) N,N'-Dicyclohexytearbodiimid in 10 ml N-Methylpyrrolidon zu. Der Reaktionsansatz wird 24 h bei 60° C gerührt und dann mit Aceton ausgefällt. Die ausgefallene Substanz wird in ca. 100 ml Wasser gegeben und mit 20-40 ml Natronlauge (40 %-ig) versetzt. Man behandelt das Gel mehrmals mit Aceton und trocknet dann bei 10 mbar. Die erhaltene Masse wird in ca. 250 ml Wasser gelöst, mit Methanol ausgefällt und im Membranpumpenvakuum getrocknet. Man wiederholt diesen Vorgang und trocknet dann 24 h bei einem Druck von 10 mbar.

### Herstellungsbeispiel für Stearoylpolyacrylat

5 g Polyacrylsäure (M = 450.000) und eine Spatelspitze 4-Dimethylaminopyridin werden in 150 ml wasserfreiem N-Methylpyrrolidon bei 60° C gelöst. Dann tropft man eine Lösung von 0,566 g (2,10 mmol) Stearylamin und 0,475 g (2,30 mmol) N,N'-Dicyclohexylcarbodiimid in 10 ml N-Methylpyrrolidon zu. Der Reaktionsansatz wird 24 h bei 60° C gerührt und dann mit Aceton ausgefällt. Die ausgefallene Substanz wird in ca. 100 ml Wasser gegeben und mit 20-40m1 Natronlauge (40 %-ig) versetzt. Man behandelt das Gel mehrmals mit Aceton und trocknet dann bei 10 mbar. Die erhaltene Masse wird in ca. 250 ml Wasser gelöst, mit Methanol ausgefällt und im Membranpumpenvakuum getrocknet. Man wiederholt diesen Vorgang und trocknet dann 24 h bei einem Druck von 10 mbar.

### Herstellungsbeispiel für PEG-800 diglycyrrhetinylstearat

10 g Glycyrrhetinylstearat werden mit 10 g K₂CO₃ (wasserfrei) in 50 ml SOCl₂ ca. 1 h am Rückfluß erhitzt. Überschüssiges SOCl₂ wird im Wasserstrahlvakuum abgezogen, der Rückstand in 150 ml siedendem Hexan aufgenommen und heiß abfiltriert. Das Filtrat wird bis zur Trockene eingedampft und das Produkt für 3h an der Ölpumpe getrocknet. Das entstandene Säurechlorid wird ohne weitere Reinigung eingesetzt.

Die Umsetzung (und Aufarbeitung) mit 40 g PEG (35.000 gmol-1) erfolgt analog zur entsprechenden Vorschrift mit Cholesterylchloroformiat.

### Herstellungsbeispiel für PEG-800 diazelat

Die Umsetzung von PEG (35.000 g mol⁻¹) mit Azelainsäuredichlorid erfolgt analog der Vorschrift mit Cholesterylchloroformiat. Zur Verseifung der freien Carbonsäurechloridgruppen wird das Polymer 24 h in einer Mischung Aceton : Wasser = 95 :5 gerührt.

### Herstellungsbeispiel für PEG-800 diretinat

44 g ( 1,26 mmol) wasserfreies PEG (35000 gmol⁻¹) werden in 100ml CH₂Cl₂ abs. unter N₂- Atmosphäre 12 h bei Raumtemperatur mit 3,02 g (10 mmol) Retinsäure, 2,08 g (10 mmol) Dicyclohexylcarbodiimid und 12 mg (0.1 mmol) Dimethylaminopyridin gerührt. Zur Reinigung wird das Polymer 3 mal in je 1,5 I Diethylether, 2 mal in je 1,5 I Petrolether (Siedebereich 40 - 60° C) ausgefällt und anschließend zweimal aus ca. 1 I Aceton umkristallisiert. Das Produkt wird durch Gefriertrocknen aus Benzol von Lösungsmittelspuren befreit.

### Mit Stearinsäure modifizierter Polyvinylalkohol: Stearylpolyvinylalkohol

5 g Polyvinylalkohol (M=250000) und eine Spatelspitze 4-Dimethylaminopyridin werden in 150 ml wasserfreiem N-Methylpyrrolidon bei 60°C gelöst. Dann tropft man eine Lösung von 0,5 g Stearinsäure und 0,475 g N,N'-Dicyclohexylcarbodiimid in 10 ml N-Methylpyrrolidon zu. Der Reaktionsansatz wird 24 h bei 60°C gerührt und dann mit Aceton ausgefällt. Man fällt das Polymer 3 mal aus Methanol um. Die erhaltene Masse 48 h bei einem Druck von 10⁻² mbar getrocknet.
Ausbeute: 4,2 g

### Copolymer aus Methycrylsäureglucosamid und Cholestylmethacrylat

Die Lösung von 5 g (20,2 mmol) Methacrylsäureglucosamid, 0,046 g (0,1 mmol) Cholesterylmethacrylat und 7 mg AIBN in 50 ml Tetrahydrofuran und 15 ml Wasser (deionisiert) wird 48 h bei 60°C gerührt. Der Ansatz wird aus 700 ml Aceton gefällt. Das im Membranpumpenvakuum getrocknete Produkt wird in ca. 50 ml Wasser gelöst und die unlöslichen Bestandteile abzentrifugiert. Die Lösung wird emeut gefällt und bei einem Druck von 10⁻² mbar getrocknet.
Ausbeute: 4,8 g

### Copolymer aus Polyvinylpyrrolidon und Cholestylmethacrylat

Zu 150 ml Ethanol (96 %, dest.) werden 15 g (134,94 mmol) Vinylpyrrolidon, 0,5 g (1,10 mml) Cholesterylmethacrylat und 100 mg AIBN gegeben. Die Suspension wird entgast und unter Argon 18 h bei 60°C gerührt. Der Reaktionsansatz wird aus 2 I Ether gefällt. Die Substanz wird in Chloroform gelöst und erneut aus Ether gefällt. (Vorgang 2 bis 3 mal wiederholen.) Nach dem Trocknen im Vakuum verbleiben 13,4 g.

### Cholestylmethacrylat

Zu 20 g (51,72 mmol) Cholesterin und 8 ml Triethylamin in 170 ml Dichlormethan (abs.) tropf man eine Lösung von 6 ml Methacrylsäurechlorid in 30 ml Dichlormethan. Man läßt über Nacht bei Raumtemperatur rühren. Das Lösungsmittel wird im Vakuum abdestilliert und der Rückstand dreimal aus ca. 400 ml Ethanol (95 %) umkristallisiert. Man trocknet 24 h bei einem Druck von 10⁻² mbar.
Ausbeute: 12,2 g.

### Methacrylsäureglucosamid

Zu einer Suspension von 25 g Clucosaminhydrochlorid in 100 ml Methanol (abs.) gibt man bei 4-10°C Innentemperatur 80 ml einer frisch bereiteten 1,5 M-Natriummethylatlösung. Es werden nun insgesamt 20 ml Methacrylsäurechlorid in 1 ml Portionen im Wechsel mit Natriummethylatlösung so zugegeben, daß der pH-Wert nach Zugabe der Natriummethylatlösung wieder zwischen 8 und 9 liegt.
Die Suspension wird 1,5 I Petrolether (30/70) eingetragen, der Niederschlag abgesaugt und in Membranpumpenvakuum getrocknet. Der Feststoff wird in ca. 250 ml Methanol unter Rückfluß erhitzt und heiß abgesaugt. Man läßt über Nacht im Gefrierfach stehen, saugt ab und trocknet im Vakuum: 10,1 g.

### Herstellungsbeispiele für POE-Ester (Triblockcopolymere)

In einem 1-Liter-Kolben wurden 40g Polyoxyethylen (POE; 1,1mmol, m = 35.000 g/mol) eingewogen, und während etwa 30h an der Ölpumpe unter Vakuum (9.3*10⁻⁵ bar) vorgetrocknet. Anschließend wurde das Polymer in 50ml Benzol in der Wärme zu einer dickflüssigen klaren Lösung gelöst und danach rasch in flüssigem Stickstoff eingefroren. Die gefrorene Polymerlösung wurde während etwa 45h unter Vakuum gefriergetrocknet. Das Benzol, das mit dem restlichen Wasser ein aceotropes Gemisch bildet, wurde absublimiert. Anschließend wurde das getrocknete POE in 1dI abs. Methylenchlorid unter Stickstoffatmosphäre gelöst.

Die Synthese der Triblockcopolymere A, F und H erfolgte durch einer Veresterung von POE mit einem Carbonsäurechlorid und die Synthese der Triblockcopolymere B, C, D und G erfolgte durch eine Veresterung von POE mit einer Carbonsäure.

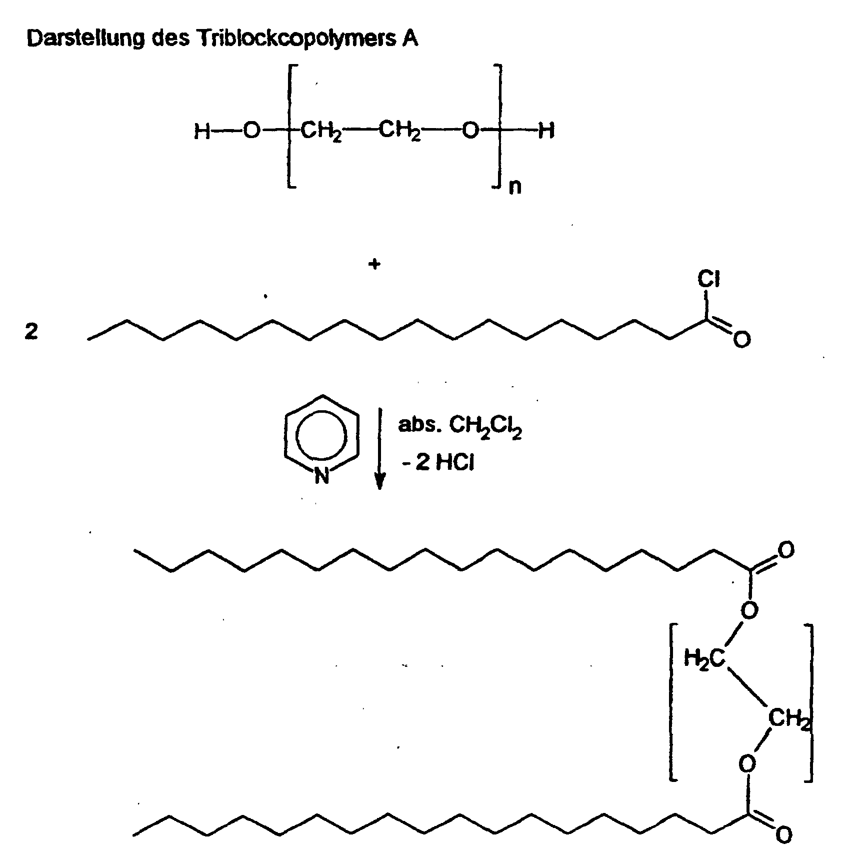

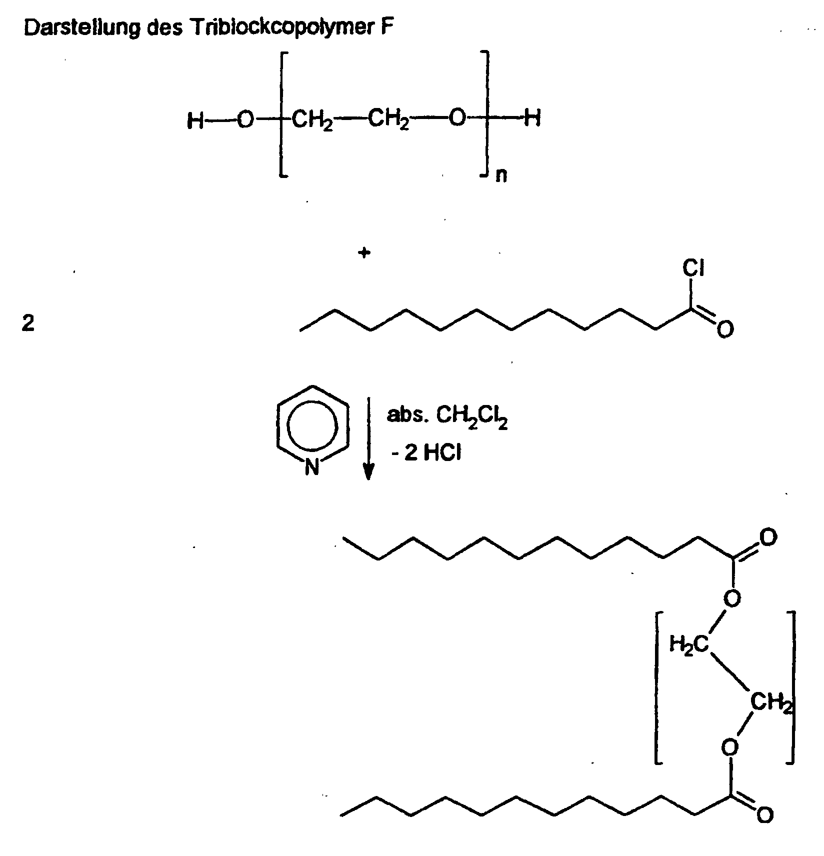

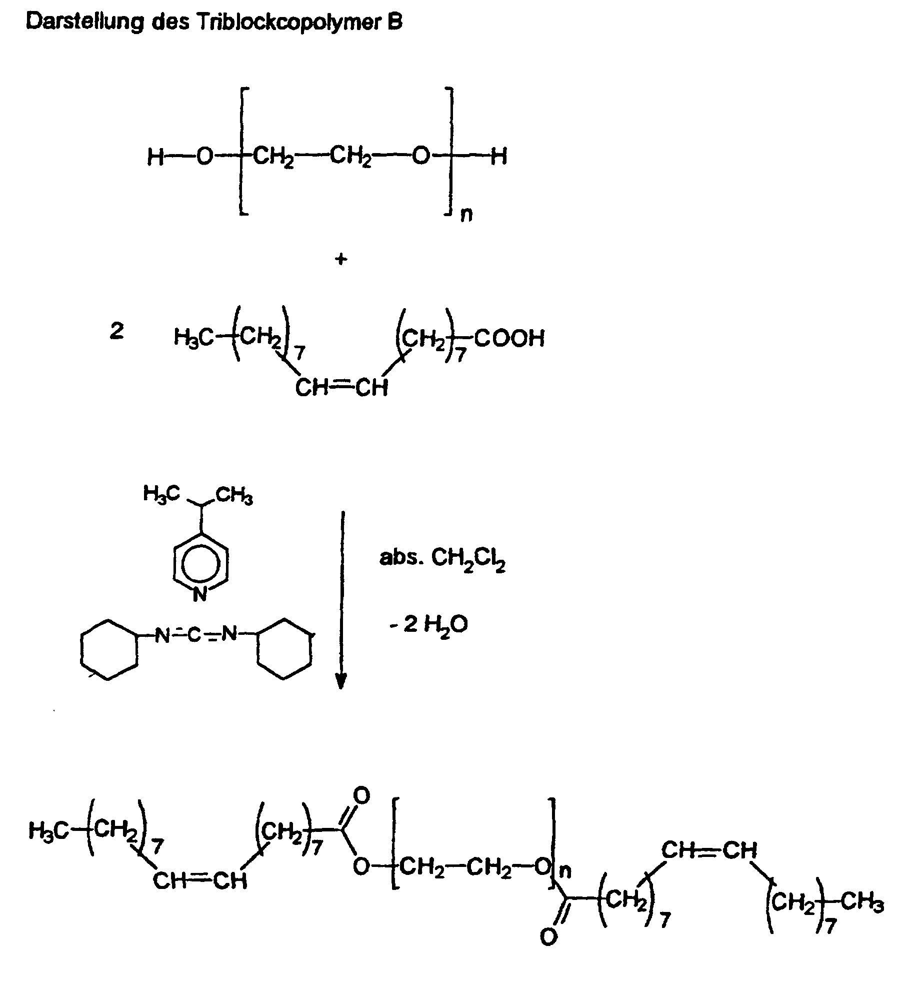

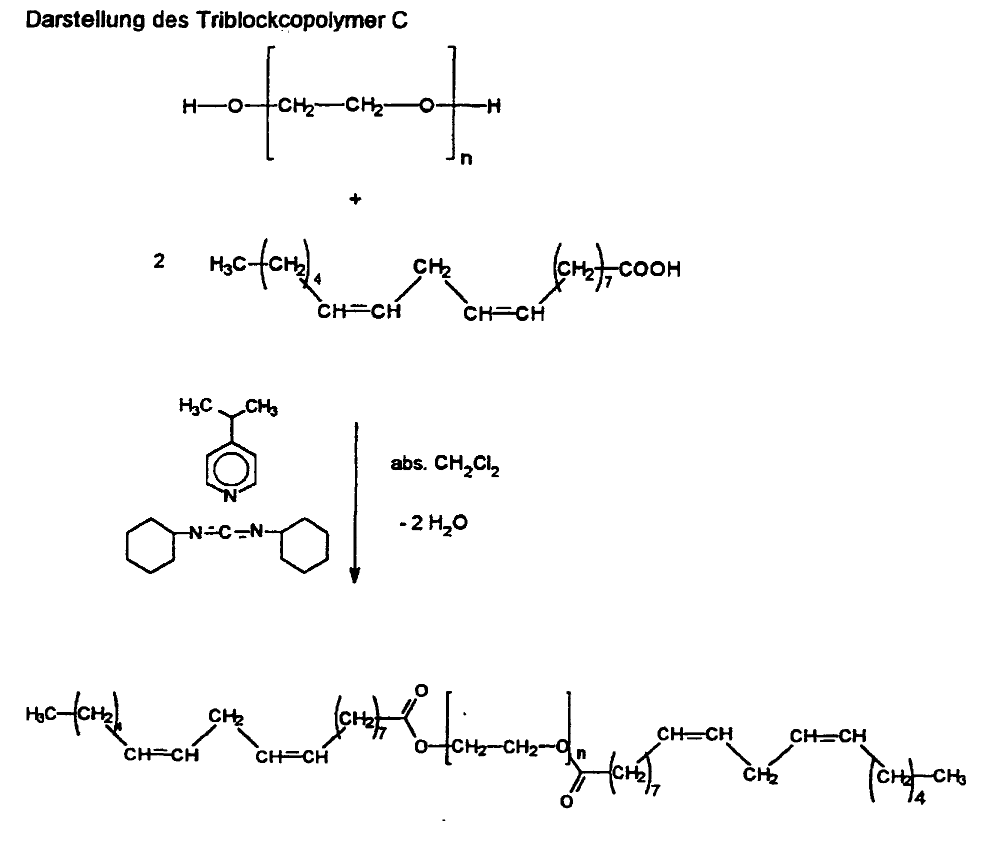

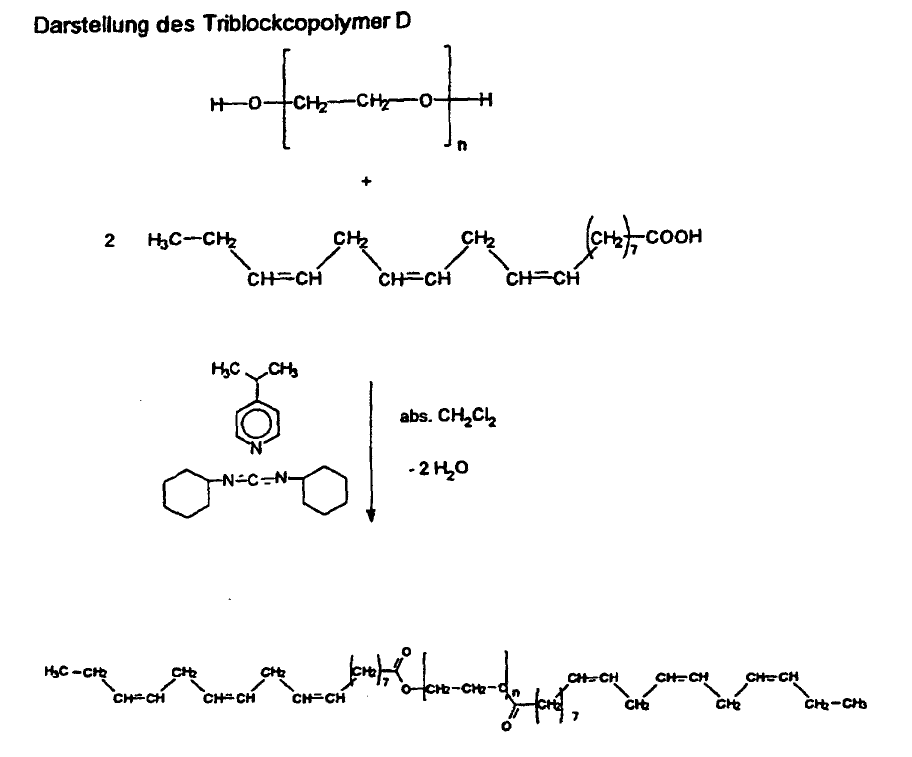

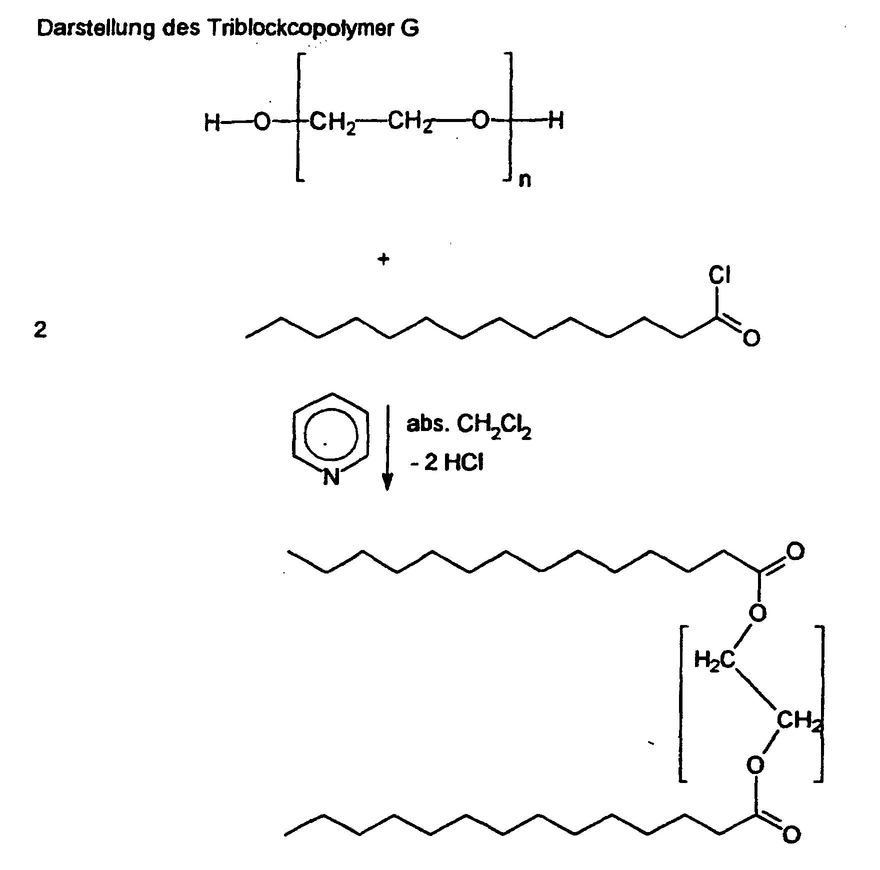

Nachdem das POE in 1dl abs. Methylenchlorid gelöst wurde, wurden unter Stickstoffatmosphäre 7g entsprechende Carbonsäure (≈30mmol, im Überschuß), 4.6g DCC und 3ml (Spatelspitze) Dimethylaminopyridin zugegeben. Das Reaktionsgemisch ließ man bei Raumtemperatur unter kräftigem Rühren während 15h reagieren.

Anschließend wurden die Lösungen aller Triblockcopolymere A-H in ein Tropftrichter versehen und drei mal in je 1,51 Diethylether und zwei mal in je 1,5I Petrolether unter langsamem zutropfen und kräftigem Rühren ausgefällt. Danach wurde der Niederschlag abfiltriert und am Rotationsverdampfer zur Trockene einrotiert. Das getrocknete Triblockcopolymer wurde in 0.5I Aceton in der Hitze gelöst bis eine klare Lösung entstand und bei -20°C während drei Stunden auskristallisiert, um die polaren Verunreinigungen zu entfernen. Darauf wurde das Polymer abfiltriert, in 1,51 Petrolether ausgefällt und nochmals in 0.51 Aceton umkristallisiert.

Anschließend wurde das Triblockcopolymer nochmals in 1.51 Diethylether und 1.51 Petrolether ausgefällt, filtriert und am Rotationsverdampfer bis zur Trockene einrotiert. Das feine weiße Pulver wurde in 1dl Benzol unter leichter Erwärmung gelöst, bis eine klare Lösung sichtbar wurde, in flüssigem Stickstoff eingefroren und während ca. 45h an der Ölpumpe unter Vakuum gefriergetrocknet.

### Beispiel 1

| **Grundlage für schäumendes Rasiergel; Gesichtpflegegel** | |
|---|---|
| Lecithin (Phospholipon 90) | 0.600 |
| Natriumlauroylactylat | 6.000 |
| PEG-150 Distearate | 1.000 |
| Dicapylylether | 5.000 |
| Glycerin | 5.000 |
| Antioxidantien, Water (Aqua) | ad 100 |

### Beispiel 2

| **Körperpflegegel** | |
|---|---|
| Polyglyceryl-3 Diisostearate | 1.600 |
| Sodium Lauroyl Lactylate | 2.800 |
| Lauryl Glucoside | 5.600 |
| Dicaprylyl Ether | 5.000 |
| Glycerin | 5.000 |
| PEG-150 Distearate | 2.000 |
| Water (Aqua) | ad 100 |

### Beispiel 3

| **Duschgel** | |
|---|---|
| Polyglyceryl-3 Diisostearate | 1.000 |
| Sodium Caproyl Lactylate | 3.100 |
| Lauryl Glucoside | 6.200 |
| Dicaprylyl Ether | 5.000 |
| Propylenglycol | 5.000 |
| PEG-300 Pentaerythrityltetraisosteararat | 2.000 |
| Water (Aqua) | ad 100 |

### Beispiel 4

| **Aftershave-Gel** | |
|---|---|
| Glycerylisostearat | 1.200 |
| Sodium Lauroyl Lactylate | 3.000 |
| Lauryl Glucoside | 6.000 |
| Dicaprylyl Ether | 5.000 |
| Butylenglycol | 5.000 |
| PEG-200 Hydrogenated Glyceryl Palmitate | 1.000 |
| Water (Aqua) | ad 100 |

### Beispiel 5

| **Haargel** | |
|---|---|
| Glyceryloleat | 2.000 |
| Sodium Lauroyl Lactylate | 6.000 |
| Butylenglycol | 4.000 |
| Dicaprylyl Ether | 5.000 |
| Glycerin | 1.000 |
| Cetylhydroxyethylcellulose | 2.000 |
| Water (Aqua) | ad 100 |

### Beispiel 6

| **Abschminkgel** | |
|---|---|
| Diglycerylmonoisostearat | 1.200 |
| Sodium Lauroyl Lactylate | 6.000 |
| Dicaprylyl Ether | 5.000 |
| Butylenglycol | 5.000 |
| PEG-230 Glyceryl Triisostearate | 2.000 |
| Water (Aqua) | ad 100 |

### Beispiel 7

| **Rasiergel-Grundlage** | |
|---|---|
| Diglycerindiisostearat | 1.200 |
| Sodium Lauroyl Lactylate | 3.000 |
| Lauryl Glucoside | 6.000 |
| Dicaprylyl Ether | 5.000 |
| Butylenglycol | 5.000 |
| PEG-800 Diglycyrrhetinylstearat | 2.000 |
| Water (Aqua) | ad 100 |

### Beispiel 8

| Reinigungsgel | |
|---|---|
| Natriumisostearoyllactylat | 2.400 |
| Sodium Lauroyl Lactylate | 4.800 |
| Dicaprylyl Ether | 5.000 |
| Proylenglycol | 3.000 |
| Stearylpolyvinylalkohol | 2.000 |
| Water (Aqua) | ad 100 |

### Beispiel 9

| **Sonnenschutzgel** | |
|---|---|
| Decaglycerylmonooleat | 5.300 |
| Glycerylmonocaprylat | 1.800 |
| Tridecylisononanoat | 3.000 |
| Eusolex 232 | 2.000 |
| Cyclomethicon | 3.000 |
| Glycerin | 5.000 |
| Cholesterylpolyacrylat | 2.000 |
| Water (Aqua) | ad 100 |

### Beispiel 10

| **Gesichtreinigungsgel** | |
|---|---|
| Glyceryl Isostearate | 1.833 |
| Polyglyceryl Laurate | 5.333 |
| Dodecylpolyacrylat | 2.000 |
| Octyl Isostearate | 3.333 |
| Cyclomethicone | 3.333 |
| Glycerin | 5.000 |
| Methylparaben | 0.250 |
| Water (Aqua) | ad 100 |

### Beispiel 11

| **Gesichtreinigungsgel** | |
|---|---|
| 2-Ethylhexylglycerinether | 1.800 |
| Natriumlauroylactylat | 5.400 |
| PEG-150 Distearate | 2.000 |
| Dicapylylether | 5.000 |
| Butylenglycol | 5.000 |
| Water (Aqua) | ad 100 |

### Beispiel 12

| **Gelgrundlage für Wundbehandlung** | |
|---|---|
| Lecithin (Epicuron 200) | 0.600 |
| Natriumlauroylactylat | 4.000 |
| PEG-150 Distearate | 2.000 |
| Dicapylylether | 5.000 |
| Glycerin | 5.000 |
| Antioxidantien, Water (Aqua) | ad 100 |

### Beispiel 13

| **Deogel** | |
|---|---|
| Natriumlauroyllactylat | 1.800 |
| Cholesterylhydroxyethylcelllose Distearate | 2.000 |
| Dicapylylether | 5.000 |
| Glycerylcaprylat | 5.400 |
| Butylenglycol | 5.000 |
| Water (Aqua) | ad 100 |

### Beispiel 14

| **Grundlage für Rasierschaum** | |
|---|---|
| Methylglucose Isostearat | 1.200 |
| Laurylglycosid | 6.000 |
| PEG-800 Dioleat | 2.000 |
| Dicapylylether | 5.000 |
| Natriumlauroyllactylat | 3.000 |
| Butylenglycol | 5.000 |
| Water (Aqua) | ad 100 |

### Beispiel 15

| **Augenmake-up-Entfernergel** | |
|---|---|
| Polyglyceryl-3 Diisostearate | 1.600 |
| Sodium Lauroyl Lactylate | 2.800 |
| Lauryl Glucoside | 5.600 |
| Dicaprylyl Ether | 5.000 |
| Glycerin | 5.000 |
| PEG-800 Chol₂ | 2.000 |
| Water (Aqua) | ad 100 |

### Beispiel 16

| | |
|---|---|
| Natriumlauroylactylat | 5.400 |
| Glycerincaprinat | 1.800 |
| PEG-800 Distearate | 2.000 |
| Dicapylylether | 5.000 |
| Butylenglycol | 5.000 |
| Water (Aqua) | ad 100 |

### Beispiel 17

| | |
|---|---|
| Polyglycerylmethylglucose Distearat | 1.200 |
| Natriumlauroylactylat | 6.000 |
| PEG-800 Distearate | 2.000 |
| Dicapylylether | 5.000 |
| Butylenglycol | 5.000 |
| Water (Aqua) | ad 100 |

### Beispiel 18

| | |
|---|---|
| Laurylglycosid | 6.400 |
| Natriumlauroylactylat | 3.200 |
| PEG-800 Distearate | 2.000 |
| Dicapylylether | 5.000 |
| Laurylglycol | 0.800 |
| Butylenglycol | 5.000 |
| Water (Aqua) | ad 100 |

### Beispiel 19

| | |
|---|---|
| 2-Ethylhexylglycerinether | 0.600 |
| Natriumlauroylsarcosinat | 6.000 |
| PEG-800 Distearate | 2.000 |
| Dicapylylether | 5.000 |
| Butylenglycol | 5.000 |
| Water (Aqua) | ad 100 |

### Beispiel 20

| | |
|---|---|
| Natriumcocoylglutamat | 0.260 |
| PEG-800 Distearate | 2.000 |
| Glycerylcaprylat | 6.960 |
| Dicapylylether | 5.000 |
| Butylenglycol | 5.000 |
| Water (Aqua) | ad 100 |

### Beispiel 21

| | |
|---|---|
| Natriumcocoylglutamat | 0.130 |
| PEG-800 Distearate | 2.000 |
| Glycerylcaprylat | 6.960 |
| Dicapylylether | 5.000 |
| Laurlglycosid | 0.260 |
| Butylenglycol | 5.000 |
| Water (Aqua) | ad 100 |

### Beispiel 22

| **Sprühgel** | |
|---|---|
| Lecithin (Phospholipon 90) | 6.100 |
| PEG-800 Distearate | 3.200 |
| Ethanol | 21.800 |
| Dicapylylether | 1.000 |
| Water (Aqua) | ad 100 |

### Beispiel 23

| **Grundlage für Aersolspray** | |
|---|---|
| Lecithin (Phospholipon 90) | 6.800 |
| PEG-800 Distearate | 2.800 |
| Ethanol | 24.000 |
| Cetearylisononanoat | 0.900 |
| Water (Aqua) | ad 100 |

### Beispiel 24

| **Grundlage für Rasiergel, Rasierschaum, Haarpflegeprodukt** | |
|---|---|
| Lecithin (Phospholipon 90) | 6.900 |
| PEG-800 Distearate | 3.000 |
| Ethanol | 23.400 |
| Caprylic/Capric Triglycerides | 1.700 |
| Water (Aqua) | ad 100 |

### Beispiel 25

| | |
|---|---|
| Glyceryl Laurate | 2.400 |
| Sucrose Laurate (Sistema) L70-C, 40%ige Lösung | 12.000 |
| Dicapylylether | 5.000 |
| Butylenglycol | 3.000 |
| PEG-150 Distearate | 1.000 |
| Water (Aqua) | ad 100 |

## Patentansprüche

1. Mikroemulsionsgele,
(a) beruhend auf Mikroemulsionen vom Typ Öl-in-Wasser, welche umfassen
- eine Ölphase, welche im wesentlichen aus schwerflüchtigen Bestandteilen zusammengesetzt ist, und eine Wasserphase
- enthaltend:
einen oder mehrere ethylenoxidfreie und propylenoxidfreie O/W-Emulgatoren und
- gewünschtenfalls ferner enthaltend einen oder mehrere W/O-Emulgatoren
- einen Emulgatorgehalt kleiner als 20 Gew.-%. bezogen auf das Gesamtgewicht der Mikroemulsion, aufweisend,
- erhältlich auf die Weise, daß man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe in ein definiertes Mischungsverhältnis zueinander setzt, so daß eine Mikroemulsion erhalten wird,
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

2. Mikroemulsionsgele gemäß Anspruch 1,
(a) beruhend auf Mikroemulsionen vom Typ Öl-in-Wasser, welche umfassen
- eine diskontiniuerliche Ölphase und eine kontinuierliche Wasserphase
- enthaltend mindestens einen ethylenoxidfreien und propylenoxidfreien O/W-Emulgator
- wobei dieser ausgewählt wird aus der Gruppe der Acyl-Lactylate, Glutamate, Sarcosinate, Isethionate, Sulfosucinate, Alaninate, Amphoacetate, Polyglycerinester, Alkylglycoside, Alkylpolyglycoside, Sorbitanester, Methylglucoseester, Ester von Hydroxysäuren und der Polyglycerinmethylglucoseester,
(b) bei welcher die Tröpfchen der diskontinuierlichen Ölphase durch eine oder mehrere Vernetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

3. Mikroemulsionsgele,
(a) beruhend auf Mikroemulsionen vom Typ Öl-in-Wasser, welche umfassen
- eine Ölphase, welche im wesentlichen aus schwerflüchtigen Bestandteilen zusammengesetzt ist,
- enthaltend:
einen oder mehrere ethylenoxidfreie und propylenoxidfreie O/W-Emulgatoren und Lecithin oder Lecithinderivate und
- gewünschtenfalls ferner enthaltend einen oder mehrere W/O-Emulgatoren
- einen Emulgatorgehalt kleiner als 20 Gew.-%, bezogen auf das Gesamtgewicht der Mikroemulsion, aufweisend,
- erhältlich auf die Weise, daß man ein Gemisch aus den Grundkomponenten, umfassend Ölphase, einen oder mehrere der erfindungsgemäßen O/W-Emulgatoren und Lecithin, gewünschtenfalls einen oder mehrere W/O-Emulgatoren, sowie gewünschtenfalls weitere Hilfs-, Zusatz- und/oder Wirkstoffe langsam mit Wasser versetzt, so daß intermediär ein Gel erhalten wird, daß bei weiterer Wasserzugabe zu einer Mikroemulsion führt,
(b) bei welcher die Tröpfchen der diskontinuierlichen Ölphase durch eine oder mehrere Vemetzersubstanzen miteinander verbunden sind, deren Moleküle sich durch mindestens einen hydrophilen Bereich auszeichnen, welcher eine Ausdehnung aufweist, die geeignet ist, den Abstand der Mikroemulsionströpfchen untereinander zu überbrücken, und durch mindestens einen hydrophoben Bereich, welcher mit den Mikroemulsionströpfchen in hydrophobe Wechselwirkung zu treten vermag.

4. Verwendung von Vernetzern gemäß Anspruch 1 zur Vernetzung oder Verdickung von Mikroemulsionen gemäß Anspruch 1 oder 3.

5. Mikroemulsionen vom Typ Öl-in-Wasser, welche umfassen
- eine Ölphase, welche im wesentlichen aus schwerflüchtigen Bestandteilen zusammengesetzt ist, und eine Wasserphase
- enthaltend:
einen oder mehrere ethylenoxidfreie und propylenoxidfreie O/W-Emulgatoren und
- gewünschtenfalls ferner enthaltend einen oder mehrere W/O-Emulgatoren
- einen Emulgatorgehalt kleiner als 20 Gew.-%, bezogen auf das Gesamtgewicht der Mikroemulsion, aufweisend,
- erhältlich auf die Weise, daß man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe in ein definiertes Mischungsverhältnis zueinander setzt, so daß eine Mikroemulsion erhalten wird.

6. Mikroemulsionen gemäß Anspruch 5 vom Typ Öl-in-Wasser, welche umfassen
- eine diskontiniuerliche Ölphase und eine kontinuierliche Wasserphase
- enthaltend mindestens einen ethylenoxidfreien und propylenoxidfreien O/W-Emulgator
- wobei dieser ausgewählt wird aus der Gruppe der Acyl-Lactylate, Glutamate, Sarcosinate, Isethionate, Sulfosucinate, Alaninate, Amphoacetate, Polyglycerinester, Alkylglycoside, Alkylpolyglycoside, Sorbitanester, Methylglucoseester, Ester von Hydroxysäuren und der Polyglycerinmethylglucoseester.

7. Mikroemulsionen vom Typ Öl-in-Wasser, welche umfassen
- eine Ölphase, welche im wesentlichen aus schwerflüchtigen Bestandteilen zusammengesetzt ist,
- enthaltend:
einen oder mehrere ethylenoxidfreie und propylenoxidfreie O/W-Emulgatoren und Lecithin oder Lecithinderivate und
- gewünschtenfalls ferner enthaltend einen oder mehrere W/O-Emulgatoren
- einen Emulgatorgehalt kleiner als 20 Gew.-%, bezogen auf das Gesamtgewicht der Mikroemulsion, aufweisend,
- erhältlich auf die Weise, daß man ein Gemisch aus den Grundkomponenten, umfassend Ölphase, einen oder mehrere der erfindungsgemäßen O/W-Emulgatoren und Lecithin, gewünschtenfalls einen oder mehrere W/O-Emulgatoren, sowie gewünschtenfalls weitere Hilfs-, Zusatz- und/oder Wirkstoffe langsam mit Wasser versetzt, so daß intermediär ein Gel erhalten wird, daß bei weiterer Wasserzugabe zu einer Mikroemulsion führt.

## Claims

1. Microemulsion gels
(a) based on microemulsions of the oil-in-water type, which comprise
- an oily phase, which is essentially composed of constituents of low volatility, and an aqueous phase
- comprising:
one or more O/W emulsifiers which is/are free from ethylene oxide and propylene oxide and
- if desired furthermore comprising one or more W/O emulsifiers
- having an emulsifier content of less than 20% by weight, based on the total weight of the microemulsion,
- obtainable by formulating a mixture of the base components, comprising the aqueous phase, the oily phase, one or more of the O/W emulsifiers according to the invention, if desired one or more W/O emulsifiers, and if desired further auxiliaries, additives and/or active compounds, in a defined mixing ratio to one another to give a microemulsion,
(b) in which the droplets of the discontinuous oily phase are joined to one another by one or more crosslinking substances, the molecules of which are distinguished by at least one hydrophilic region, which has an extension which is suitable for bridging the distance between the microemulsion droplets, and by at least one hydrophobic region, which is capable of entering into a hydrophobic interaction with the microemulsion droplets.

2. Microemulsion gels according to Claim 1,
(a) based on microemulsions of the oil-in-water type, which comprise
- a discontinuous oily phase and a continuous aqueous phase
- comprising at least one O/W emulsifier which is/are free from ethylene oxide and propylene oxide
- where the latter is chosen from the group consisting of acyl lactylates, glutamates, sarcosinates, isethionates, sulphosuccinates, alaninates, amphoacetates, polyglycerol esters, alkylglycosides, alkylpolyglycosides, sorbitan esters, methylglucose esters, esters of hydroxy acids, and polyglycerol methylglucose esters,
(b) in which the droplets of the discontinuous oily phase are joined to one another by one or more crosslinking substances, the molecules of which are distinguished by at least one hydrophilic region, which has an extension which is suitable for bridging the distance between the microemulsion droplets, and by at least one hydrophobic region, which is capable of entering into a hydrophobic interaction with the microemulsion droplets.

3. Microemulsion gels,
(a) based on microemulsions of the oil-in-water type, which comprise
- an oily phase, which is essentially composed of constituents of low volatility,
- comprising:
one or more O/W emulsifiers which is/are free from ethylene oxide and propylene oxide, and lecithin or lecithin derivatives and
- if desired furthermore comprising one or more W/O eniulsifiers
- having an emulsifier content of less than 20% by weight, based on the total weight of the microemulsion,
- obtainable by slowly adding water to a mixture of the base components, comprising the oily phase, one or more of the O/W emulsifiers according to the invention and lecithin, if desired one or more W/O emulsifiers, and if desired further auxiliaries, additives and/or active compounds, such that an intermediate gel is formed which, on the addition of more water, leads to a microemulsion,
(b) in which the droplets of the discontinuous oily phase are joined to one another by one or more crosslinking substances, the molecules of which are distinguished by at least one hydrophilic region, which has an extension which is suitable for bridging the distance between the microemulsion droplets, and by at least one hydrophobic region; which is capable of entering into a hydrophobic interaction with the microemulsion droplets.

4. Use of crosslinkers according to Claim 1 for crosslinking or thickening microemulsions according to Claim 1 or 3.

5. Microemulsions of the oil-in-water type, which comprise
- an oily phase, which is essentially composed of constituents of low volatility, and an aqueous phase
- comprising:
one or more O/W emulsifiers which is/are free from ethylene oxide and propylene oxide and
- if desired furthermore comprising one or more W/O emulsifiers
- having an emulsifier content of less than 20% by weight, based on the total weight of the microemulsion,
- obtainable by formulating a mixture of the base components, comprising the aqueous phase, the oily phase, one or more of the O/W emulsifiers according to the invention, if desired one or more W/O emulsifiers, and if desired further auxiliaries, additives and/or active compounds, in a defined mixing ratio to one another to give a microemulsion.

6. Microemulsions according to Claim 5 of the oil-in-water type, which comprise
- a discontinuous oily phase and a continuous aqueous phase
- comprising at least one O/W emulsifier which is/are free from ethylene oxide and propylene oxide
- where the latter is chosen from the group consisting of acyl lactylates, glutamates, sarcosinates, isethionates, sulphosuccinates, alaninates, amphoacetates, polyglycerol esters, alkylglycosides, alkylpolyglycosides, sorbitan esters, methylglucose esters, esters of hydroxy acids, and polyglycerol methylglucose esters.

7. Microemulsions of the oil-in-water type, which comprise
- an oily phase, which is essentialiy composed of constituents of low volatility,
- comprising:
one or more O/W emulsifiers which is/are free from ethylene oxide and propylene oxide, and lecithin or lecithin derivatives and
- if desired furthermore comprising one or more W/O emulsifiers
- having an emulsifier content of less than 20% by weight, based on the total weight of the microemulsion,
- obtainable by slowly adding water to a mixture of the base components, comprising the oily phase, one or more of the O/W emulsifiers according to the invention and lecithin, if desired one or more W/O emulsifiers, and if desired further auxiliaries, additives and/or active compounds, such that an intermediate gel is formed which, on the addition of more water, leads to a microemulsion.

## Revendications

1. Gels de micro-émulsions,
(a) reposant sur des micro-émulsions du type huile dans l'eau, qui comprennent :
- une phase huile, qui est composée pour l'essentiel de constituants peu volatils, et une phase eau contenant :
- un ou plusieurs émulsionnant (s) huile dans l'eau sans oxyde d'éthylène et sans oxyde de propylène
et
- contenant en outre, si on le souhaite, un ou plusieurs émulsionnant(s) eau dans l'huile
- présentant une teneur en émulsionnant(s) inférieure à 20% en poids, par rapport au poids total de la micro-émulsion,
- que l'on peut obtenir en regroupant, dans un rapport de mélange défini, un mélange constitué des composants de base, comportant une phase eau, une phase huile, un ou plusieurs des émulsionnants huile dans l'eau selon l'invention, si on le souhaite un ou plusieurs émulsionnants eau dans l'huile ainsi que, si on le souhaite, d'autres additifs, adjuvants et/ou substances actives, afin d'obtenir une micro-émulsion,
(b) dans lesquels les gouttelettes de la phase huile discontinue sont reliées entre elles par une ou plusieurs substances(s) réticulante(s), dont les molécules sont **caractérisées par** au moins une partie hydrophile, qui présente une extension qui convient pour ponter l'écart des gouttelettes de micro-émulsion entre elles et par au moins une partie hydrophobe qui peut entrer en interaction hydrophobe avec les gouttelettes de micro-émulsion.

2. Gels de micro-émulsions selon la revendication 1,
(a) reposant sur des micro-émulsions du type huile dans l'eau, qui comprennent :
- une phase huile discontinue et une phase eau continue
- contenant au moins un émulsionnant huile dans l'eau sans oxyde d'éthylène et sans oxyde de propylène
- ce dernier étant choisi dans le groupe des lactylates d'acyle, glutamates, sarcosinates, iséthionates, sulfosuccinates, alaninates, amphoacétates, esters de polyglycérol, alkylglycosides, alkylpolyglycosides, esters de sorbitan, esters de méthylglucose, esters d'hydroxyacides et esters de méthylglucose de polyglycérol,
(b) dans lesquels les gouttelettes de la phase huile discontinue sont reliées entre elles par une ou plusieurs substances(s) réticulantes(s) dont les molécules sont **caractérisées par** au moins une partie hydrophile qui présente une extension qui convient pour ponter l'écart des gouttelettes de micro-émulsion entre elles, et par au moins une partie hydrophobe qui peut entrer en interaction hydrophobe avec les gouttelettes de micro-émulsion.

3. Gels de micro-émulsions,
(a) reposant sur des micro-émulsions du type huile dans l'eau, qui comprennent :
- une phase huile, qui est composée pour l'essentiel de constituants peu volatils contenant :
- un ou plusieurs émulsionnant(s) huile dans l'eau sans oxyde d'éthylène et sans oxyde de propylène et de la lécithine ou des dérivés de la lécithine, et
- contenant en outre, si on le souhaite, un ou plusieurs émulsionnant(s) eau dans l'huile
- présentant une teneur en émulsionnant(s) inférieure à 20% en poids, par rapport au poids total de la micro-émulsion,
- que l'on peut obtenir en ajoutant lentement de l'eau à un mélange constitué des composants de base, comportant une phase huile, un ou plusieurs des émulsionnants huile dans l'eau, selon l'invention, et de la lécithine, si on le souhaite, un ou plusieurs émulsionnant(s) eau dans l'huile, ainsi que, si on le souhaite, d'autres additifs, adjuvants et/ou substances actives, afin d'obtenir, de manière intermédiaire, un gel qui donne, après addition d'eau supplémentaire, une micro-émulsion,
(b) dans lesquels les gouttelettes de la phase huile discontinue sont reliées entre elles par une un plusieurs substance(s) réticulante(s) dont les molécules sont **caractérisées par** au moins une partie hydrophile qui présente une extension qui convient pour ponter l'écart des gouttelettes de micro-émulsions entre elles et par au moins une partie hydrophobe qui peut entrer en interaction hydrophobe avec les gouttelettes de micro-émulsion.

4. Utilisation d'agents de réticulation selon la revendication 1 pour la réticulation ou l'épaississement de micro-émulsions selon la revendication 1 ou 3.

5. Micro-émulsions du type huile dans l'eau, qui comportent :
- une phase huile, qui est composée essentiellement de constituants peu volatils, et une phase eau contenant :
- un ou plusieurs émulsionnant(s) huile dans l'eau sans oxyde d'éthylène et sans oxyde de propylène,
et
- contenant eh outre, si on le souhaite, un ou plusieurs émulsionnant(s) eau dans l'huile
- présentant une teneur en émulsionnant(s) inférieure à 20% en poids, par rapport au poids total de la micro-émulsion,
- que l'on peut obtenir en réunissant, dans un rapport de mélange défini, un mélange constitué des composants de base, comportant une phase eau, une phase huile, un ou plusieurs des émulsionnants huile dans l'eau selon l'invention, si on le souhaite un ou plusieurs des émulsionnants eau dans l'huile, ainsi que, si on le souhaite, d'autres adjuvants, additifs et/ou substances actives, afin d'obtenir une micro-émulsion.

6. Micro-émulsions selon la revendication 5 du type huile dans l'eau, qui comportent :
- une phase huile discontinue et une phase eau continue,
- contenant au moins un émulsionnant huile dans l'eau sans oxyde d'éthylène et sans oxyde de propylène,
- ce dernier étant choisi dans le groupe des lactylates d'acyle, glutamates, sarcosinates, iséthionates, sulfosuccinates, alaninates, amphoacétates, esters de polyglycérol, alkylglycosides, alkylpolyglycosides, esters de sorbitan, esters de méthylglucose, esters d'hydroxyacides et esters de méthylglucose de polyglycérol.

7. Micro-émulsions du type huile dans l'eau, qui comportent :
- une phase huile, qui est composée essentiellement de constituants peu volatils contenant :
- un ou plusieurs émulsionnant(s) huile dans l'eau sans oxyde d'éthylène et sans oxyde de propylène et de la lécithine ou des dérivés de la lécithine, et
- contenant en outre, si on le souhaite, un ou plusieurs émulsionnant(s) eau dans l'huile,
- présentant une teneur en émulsionnant (s) inférieure à 20% en poids, par rapport au poids total de la micro-émulsion,
- que l'on peut obtenir en ajoutant de l'eau lentement à un mélange constitué des composants de base comprenant une phase huile, un ou plusieurs des émulsionnants huile dans l'eau selon l'invention et de la lécithine, si on le souhaite, un ou plusieurs émulsionnant(s) eau dans l'huile ainsi que, si on le souhaite, d'autres adjuvants, additifs et/ou substances actives, afin d'obtenir, de manière intermédiaire, un gel qui donne, par addition d'eau supplémentaire, une micro-émulsion.
